(19) Europäisches Patentamt / European Patent Office / Office européen des brevets

(11) **EP 1 392 621 B1**

(12) # EUROPÄISCHE PATENTSCHRIFT

(45) Veröffentlichungstag und Bekanntmachung des Hinweises auf die Patenterteilung:
**13.10.2010 Patentblatt 2010/41**

(21) Anmeldenummer: **02754628.2**

(22) Anmeldetag: **05.06.2002**

(51) Int Cl.:
*C07B 63/00* (2006.01)   *C07C 67/56* (2006.01)
*C07C 69/587* (2006.01)   *C07C 51/47* (2006.01)
*C07C 57/03* (2006.01)

(86) Internationale Anmeldenummer:
**PCT/EP2002/006158**

(87) Internationale Veröffentlichungsnummer:
**WO 2002/098826 (12.12.2002 Gazette 2002/50)**

(54) **FESTPHASEN-EXTRAKTIONSVERFAHREN ZUR GEWINNUNG VON HOCHREINEN UNGESÄTTIGTEN VERBINDUNGEN BZW. DERIVATE DIESER VERBINDUNGEN**

SOLID PHASE EXTRACTION METHOD FOR OBTAINING HIGH-PURITY UNSATURATED COMPOUNDS OR DERIVATIVES OF SAID COMPOUNDS

PROCEDE D'EXTRACTION EN PHASE SOLIDE POUR LA PRODUCTION DE COMPOSES INSATURES TRES PURS OU DE DERIVES DE CES COMPOSES

(84) Benannte Vertragsstaaten:
**AT BE CH CY DE DK ES FI FR GB GR IE IT LI LU MC NL PT SE TR**

(30) Priorität: **05.06.2001 DE 10127111**

(43) Veröffentlichungstag der Anmeldung:
**03.03.2004 Patentblatt 2004/10**

(73) Patentinhaber: **Nutrinova**
**Nutrition Specialties & Food Ingredients GmbH**
**65926 Frankfurt am Main (DE)**

(72) Erfinder:
• **FABRITIUS, Dirk**
  **60529 Frankfurt am Main (DE)**
• **REIMANN, Silke**
  **91522 Ansbach (DE)**
• **NEUMANN, Doreen**
  **65719 Hofheim (DE)**
• **MINÖR, Daniel**
  **65933 Frankfurt am Main (DE)**

(74) Vertreter: **Luderschmidt, Schüler & Partner**
**Patentanwälte**
**John-F.-Kennedy-Strasse 4**
**65189 Wiesbaden (DE)**

(56) Entgegenhaltungen:
FR-A- 2 650 274    US-A- 4 189 442
US-A- 4 721 584

• **ADLOF R O ET AL: "THE ISOLATION OF OMEGA-3 POLYUNSATURED FATTY ACIDS AND METHYL ESTERS OF FISH OILS BY SILVER RESIN CHROMATOGRAPHY" JOURNAL OF THE AMERICAN OIL CHEMISTS' SOCIETY, AMERICAN OIL CHEMISTS' SOCIETY. CHAMPAIGN, US, Bd. 62, Nr. 11, November 1985 (1985-11), Seiten 1592-1595, XP001108995 ISSN: 0003-021X in der Anmeldung erwähnt**
• **DEJARLAIS W J ET AL: "ACETONITRILE AS ELUENT IN SILVER RESIN COLUMN CHROMATOGRAPHY" JOURNAL OF THE AMERICAN OIL CHEMISTS' SOCIETY, AMERICAN OIL CHEMISTS' SOCIETY. CHAMPAIGN, US, Bd. 60, Nr. 5, Mai 1983 (1983-05), Seiten 975-978, XP001108997 ISSN: 0003-021X in der Anmeldung erwähnt**
• **ADLOF R O ET AL: "PARTIAL ARGENTATION RESIN CHROMATOGRAPHY (PARC): I. EFFECT OF PERCENT SILVER ON ELUTION AND SEPARATION OF METHYL OCTADECADIENOATE ISOMERS" JOURNAL OF THE AMERICAN OIL CHEMISTS' SOCIETY, AMERICAN OIL CHEMISTS' SOCIETY. CHAMPAIGN, US, September 1980 (1980-09), Seiten 273-275, XP001108996 ISSN: 0003-021X in der Anmeldung erwähnt**

**Beschreibung**

Die vorliegende Erfindung betrifft ein neuartiges Extraktionsverfahren zum Gewinnen einer oder mehrerer ungesättigter, gegebenenfalls derivatisierter Verbindungen aus Gemischen von diesen mit anderen weniger stark gesättigten Komponenten, z.B. die Gewinnung von mehrfach ungesättigten Fettsäuren oder deren Derivaten aus Gemischen mit gesättigten und/oder weniger stark ungesättigten, gegebenenfalls derivatisierten Fettsäuren, durch selektive Komplexierung an einen partiell oder vollständig mit Silberionen beladenen Kationentauscher und anschließender Dekomplexierung.

Hochungesättigte langkettige Fettsäuren (Polyunsaturated Fatty Acids; PUFAs) stellen essentielle Fettsäuren des menschlichen Stoffwechsels dar. PUFA können in zwei große Gruppen unterteilt werden. Neben der Gruppe der ω-6 PUFAs, die ausgehend von Linolsäure formuliert wird, gibt es die Gruppe der ω-3 PUFAs, die ausgehend von der α-Linolensäure aufgebaut wird.

PUFAs sind wichtige Bausteine der Zellmembranen, der Retina und der Hirnhaut und Vorläufer für wichtige Hormone, beispielsweise Prostaglandine, Thromboxane und Leukotriene.

Neben der Funktion als Bausteine hat sich im Laufe der letzten Jahre immer mehr gezeigt, dass PUFAs direkt vielfältige positive Wirkungen auf den menschlichen Organismus bzw. Erkrankungen haben.

Eine Vielzahl von klinischen Studien haben gezeigt, dass PUFAs bei z.B. Krebs, rheumatischer Arthritis, Bluthochdruck und Neurodermitis und vielen anderen Erkrankungen einen wichtigen Beitrag zur Heilung oder Linderung leisten können. Diese Erkenntnisse waren ursächlich dafür verantwortlich, dass internationale Institutionen und Behörden Empfehlungen ausgesprochen haben, die die tägliche Aufnahmemenge von PUFAs regeln.

PUFAs können vom Menschen nicht *de-novo* synthetisiert werden, da ihnen die Enzymsysteme fehlen, die eine Doppelbindung in die Kohlenstoffkette an Positionen > C9 einführen können (fehlende Δ12-Desaturase). Erst durch die Zufuhr von sogenannten Vorläufer-Fettsäuren (Precursoren, z.B. α-Linolensäure) über die Nahrung ist der Mensch in der Lage, hochungesättigte Fettsäuren zu synthetisieren. Ob diese Menge jedoch ausreicht, um den Bedarf an hochungesättigten Fettsäuren zu decken, ist umstritten.

Der allergrößte Teil der essentiellen Fettsäuren wird durch die Nahrung aufgenommen. Insbesondere Pflanzenöle sind angereichert mit ω-6 Fettsäuren, (beispielsweise enthält Nachtkerzenöl γ-Linolensäure (GLA)), jedoch nur bis zu einer Kettenlänge von C18, und Fischöle bzw. Öle aus Mikroorganismen mit ω-3 Fettsäuren (z.B. enthält Lachsöl Eicosapentaensäure (EPA) und Docosahexaensäure (DHA)). Grundsätzlich stellen Fischöle und Öle aus Mikroorganismen die einzige kommerzielle Quelle für hochungesättigte Fettsäuren dar. Im Allgemeinen ist jedoch der Gehalt an der erwünschten PUFA gering und diese liegt in einer Mischung vor, wobei antagonistisch wirkende PUFAs ebenfalls enthalten sein können. Um die empfohlene tägliche Dosis an PUFAs zu sich zu nehmen, muß also eine hohe Ölmenge aufgenommen werden. Insbesondere trifft das auf solche Patienten zu, die hohe Dosen von PUFAs zu sich nehmen müssen (beispielsweise bei Cystischer Fibrose). Um eine möglichst gezielte Wirkung der einzelnen PUFAs zu erreichen, müssen angereicherte bzw. hochreine PUFAs eingesetzt werden. Es besteht daher im Stand der Technik ein hoher Bedarf an hochreinen PUFAs.

Der Einsatz Silber-beladener Kationentauscher zur Aufreinigung und Trennung ungesättigter Fettsäuren in analytischen Chromatographie-Systemen (Säulenchromatographie) wurde bereits beschrieben.

Dabei werden sowohl HPLC- [Adlof, R. O. and E.A. Emken (1985): The isolation of omega-3 polyunsaturated fatty acids and methyl esters of fish oil by silver resin chromatography. JAOCS, 62(11), 1592-1595; Christie, W.W. (1987): A stable silver loaded column for the separation of lipids by high performance liquid chromatography. J. of High resolution Chromatography & Chromatography Communications, 10, 148-150], SFC- [Kadota, Yasuhiko; Tanaka, Isao; Ohtsu, Yutaka; Yamaguchi, Michihiro (1998): Separation of polyunsaturated fatty acids by chromatography using a silver-loaded spherical clay. II Industrial-scale preparation of high purity DHA. Nihon Yyukagakkaaishi, 47(4), 351-357] und Säulenchromatographie-Systeme [Nieto, S.; Ana M Cordoba; J. Sanhueza and A. Velenzueela (1997): Obtention of highly purified fractions of eicosapentaenoic acid and docosahexaenoic acid from sardine oil by silver-resin chromatography: A semi-preparative procedure. Grasas y Aceites, 48(4), 197-199; Belarbi, El Hassan; Emilio Molina and Yusuf Chisti (2000): A process for high yield and scalable recovery of high purity eicosapentaenoic acid esters from microalgae. Enzyme and Microbial Technology, 26, 516-529] mit Kieselgelen als auch Ionenaustauschern eingesetzt. Dies trifft insbesondere für analytische Verfahren zu.

Eine weitere Chromatographiemethode stellt die sogenannte Liganden-Austausch-Chromatographie (Ligand-Exchange Chromatography; LEC) dar. Diese wurde besonders in Systemen eingesetzt, bei denen keine PUFAs getrennt werden sollten [Pyell, U., S. Schober and G. Stork (1997): Ligand-exchange chromatographic separation of polycyclic aromatic hydrocarbons and polycyclic aromatic sulfur heterocycles on a chelating silic gel loaded with palladium (II) or silver (I) cations. Fresenius J Anal Chem., 359, 538-541; Janak, K., M. Demirbueker, I. Haegglund and L.G. Blomberg (1992): Modifications of poly(methyl-3-propylthiol)siloxane to give stationary phases for open tubular supercritical fluid chromatography. Chromatographia. 34(5-8), 335-341].

Silberbeladene Kationenaustauschersysteme finden in der Anmeldeschrift JP 45021376 Verwendung, wobei Dowex® 50 als Chromatographiematerial für die Aufreinigung von Ethylestergemischen ausgehend von Nachtkerzenöl beschrieben wird. Es gelang, eine reine GLAEE-Fraktion (GLAEE = γ-Linolensäureethylester) in 78%-iger Ausbeute zu isolieren.

Bei allen Systemen tritt das Problem auf, dass sich die hochungesättigten Fettsäuren nur extrem langsam und schlecht von der Säule eluieren lassen (Adlof, R.O.; H. Rakoff and E. A. Emken (1980): Partial Argentation Resin Chromatography (PARC): I. Effect of Percent Silver on Elution and Separation of Methyl Octadecadienoate Isomers. JAOCS, 9 , 273-275). Dabei werden hohe Mengen Lösungsmittel verbraucht. Weiterhin sind Fraktionierungsschritte erforderlich, um die aufgereingten Produktfraktionen zu separieren.

Erst die Anwendung von reinem Acetonitril (ACN) oder Gemischen mit Acetonitril führte zu einer akzeptablen Elutionszeit (DeJarlais, W. J.; R. O. Adlof and E. A. Emken (1983): Acetonitrile as Eluent in Silver Resin Column Chromatography. JAOCS, 60(5), 975-978. Dabei werden in der Regel Gradientensysteme verwendet, die die Regeneration der Lösungsmittel erschweren.

Für die technische Anwendung sind große Mengen Acetonitril aufgrund des hohen Preises und der toxikologischen Bedenklichkeit (Acetonitril kann Blausäure (HCN) enthalten) denkbar ungeeignet.

Auch das *Scale up* der Chromatographieverfahren und damit die technische Anwendung ist aufgrund der zu verwendenden Säulendimensionen nur begrenzt möglich.

Adlof et al (1980, supra) haben teilversilberte Kationentauscher hergestellt, die eine Elution von zweifach ungesättigten Fettsäureestern mit Alkoholen als Elutionsmitteln ermöglichten. Höher ungesättigte Fettsäuren wurden nicht eingesetzt. Es ist jedoch davon auszugehen, dass hier ebenfalls eine Elution ohne Acetonitril nicht möglich ist.

Es wird berichtet, dass ausgehend von Fischölen für die Produktion mittels SFC Kosten von $ 550/kg DHAEE entstehen [Alkio, M.; C. Gonzalez; M. Jäntti and O. Altonen (2000): Purification of polyunsaturated fatty acid esters from Tuna oil with supercritical fluid chromatography. JAOCS, 77 (3), 315-321]. Dabei wird von einer Produktionsrate von ca. 0,5 g DHAEE pro kg stationäre Phase und Stunde gesprochen. Dies entspricht einer Beladung (DHAEE/stationäre Phase) von 0,05%. Die Investitionskosten der SFC-Anlage belaufen sich auf $ 2 Millionen.

Ebenfalls bekannt ist ein SFC-Produktionsverfahren mit einer silberbeladenen Matrix ausgehend von Fischöl, bei dem DHAEE für $ 4000/kg hergestellt werden kann [Tanaka, Isao (1996): Supercritical chromatography facilitates fatty acid production. Chem. Eng. April, 19-21].

Yamamura et al (Yamamura, R. and Y. Shimomura (1997): Industrial highperformance liquid chromatography purification of docosahexaenoic acid ethyl ester and docosapentaenoic acid ethyl ester from single cell oil. JAOCS, 74 (11), 1435-1440) berichten von einem HPLC-Produktionsverfahren ausgehend von einem Single-Cell Oil, bei dem die Herstellungskosten nicht angegeben wurden. Die Produktivität an DHAEE betrug dabei 0,1 kg/h. Betrachtet man die eingesetzte Säule (ca. 160 kg Kieselgel) ergibt sich eine Produktivität von 0,0006 kg pro kg stationäre Phase pro Stunde. Dies entspricht einer Beladung von 0,06%. Als Lösungsmittel wurde giftiges Methanol verwendet.

Es wird klar, dass chromatographische Systeme eine Vielzahl von Problemen für die Aufreinigung von PUFAs bergen und deshalb nicht geeignet sind, um hochungesättigte Fettsäuren im größeren Maßstab herzustellen (Zhou, Dequan and Xuebing Xu (2000): Enzymatic enrichment of Long-chain Polyunsaturated Fatty Acids from Fish Oils. Shipin Kexue (Bejing), 21(12), 188-194. Teramoto, M.; H. Matsuyama; N. Ohnishi; S. Uwagawa and K. Nakai (1994): Extraction of ethyl and methyl esters of polyunsaturated fatty acids with aqueous silver nitrate solutions. Ind. Eng. Chem. Res., 33, 341-345).

Auf der anderen Seite könnten milde Extraktionsverfahren erhebliche Vorteile für die Gewinnung von PUFAs aus Gemischen mit anderen Substanzen bieten.

Erste Ansätze solcher wässriger Extraktionssysteme mit Silbernitrat wurden untersucht (Suzuki, T.; S. Kikuchi; K. Nakano; S. Kato and K. Nagahama (1993): Supercritical fluid extraction of polyunsaturated fatty acid ethyl esters from aqueous silver nitrate solution. Bioseparation, 3, 197-204, Teramoto et al 1994). Kato et al (S. Kato, N. Kunio, N. Hidetomi and N. Kaoru (1994): *Separation and recovery of polyunsaturated fatty acids by emulsion film method.* JP06279781) untersuchten die Verwendung von Öl in Wasser-Emulsionen, wobei lediglich eine Trennung der Gesamt-PUFA Fraktion berichtet wurde. Dabei ist jedoch die Verwendung von gesundheitlich bedenklichem Silbernitrat hinderlich für eine technische Anwendung. Weiterhin ist in wässriger Umgebung eine Gefahr der Oxidation der PUFAs besonders hoch, die insbesondere durch das Vorhandensein von Silbernitrat noch beschleunigt werden kann. Die Handhabung von Silbernitrat erweist sich weiterhin als sehr schwierig, da Silbernitrat relativ schnell zu Silberoxid oxidiert werden kann und im Allgemeinen als instabil angesehen wird. Festphasen-Extraktionssysteme ohne Silber, die bereits beschrieben wurden, zeigen keine ausreichende Selektivität bei der Trennung von PUFAs (Wilson, R.; J. R. Henderson; I. Burkow and J. R. Sargent (1993): The enrichment of n-3 polyunsaturated fatty acids, using aminopropyl solid phase extraction columns. Lipids, 28(1), 51-54). Festphasen-Extraktionsverfahren mit Silber zur Auftrennung von PUFAs, insbesondere ohne die Verwendung von Acetonitril, sind den Anmeldern nicht bekannt.

Weiterhin wurde eine Aufreinigung durch erleichterte Diffusionen durch Silberbeladene Membranen versucht [Shibaki, A.; Y. Irimoto; K. Saito; K. Sugita; T. Baba; I. Honjyo; S. Moriyama and T. Sugo (1999): Selective Binding of Docosahexaenoic acid ethyl ester to a silver ion-loaded porous hollow-fiber membrane. JAOCS, 76(7), 771-775].

Aufgrund der Richtlinie 88/344/EWG vom 13. Juni 1988 zur Angleichung der Rechtsvorschriften der Mitgliedstaaten über Extraktionsmittel, die bei der Herstellung von Lebensmitteln und Lebensmittelzutaten zu beachten ist, muß bei der Gewinnung von DHA für den Bereich Lebensmittel die Verwendung von Acetonitril zwingend ausgeschlossen werden.

Die US 4,721,584 offenbart ein Verfahren zur Auftrennung von ungesättigten Fettsäuren, bei dem ein Aluminosilikatzeolith zum Einsatz kommt, der mit diversen Kationen, wie z. B. Silberionen beladen sein kann. Die Ablösung von ungesättigten Fettsäuren, die an den kationisch beladenen Aluminosilikatzeolith komplexieren, erfolgt durch Desorption der Fettsäuren mittels polarer Lösungsmittel.

Angesichts des genannten Stands der Technik lag der vorliegenden Erfindung daher die Aufgabe zugrunde, ein neuartiges Verfahren zur Gewinnung von ungesättigten Verbindungen aus Gemischen mit anderen Substanzen, beispielsweise anderen organischen gesättigten oder weniger stark ungesättigten Verbindungen zur Verfügung zu stellen.

Dieses Verfahren sollte dabei eine bessere als im Stand der Technik beschriebene, quantitativ ausreichende Aufreinigung ermöglichen, und eine Vereinfachung des Verfahrens und eine ökonomische Ausgestaltung desselben erlauben. Außerdem sollte das Verfahren selektiv sein, um beispielsweise die Trennung strukturell nur wenig unterschiedlicher ungesättigter Verbindungen voneinander zu erlauben.

Eine weitere Aufgabe der vorliegenden Erfindung war es, ein neuartiges Verfahren zum Gewinnen von ungesättigten Fettsäuren oder von deren Derivaten aus Gemischen mit anderen Substanzen zur Verfügung zu stellen.

Dieses Verfahren sollte dabei eine bessere als im Stand der Technik beschriebene, quantitativ ausreichende Aufreinigung von PUFAs ermöglichen, und eine Vereinfachung des Verfahrens und eine ökonomische Ausgestaltung desselben erlauben. Außerdem sollte das Verfahren selektiv sein, um beispielsweise die Trennung strukturell nur wenig unterschiedlicher ungesättigter Fettsäuren bzw. deren Derivate wie beispielsweise DHAEE/DPAEE zu ermöglichen.

Vorzugsweise soll das erfindungsgemäße Verfahren zum Einsatz im Lebensmittelbereich geeignet sein, und daher beispielsweise die Reinigung von PUFAs aus Gemischen ohne die Verwendung von Acetonitril oder anderer toxischer Lösungsmittel als Extraktionsmittel ermöglichen.

Gelöst wird diese sowie weitere, nicht explizit genannten Aufgaben, die jedoch aus den hierin einleitend diskutierten Zusammenhängen ohne weiteres ableitbar oder erschließbar sind, durch ein Verfahren nach Patentanspruch 1. Zweckmäßige Abwandlungen des erfindungsgemäßen Verfahrens werden in den auf Anspruch 1 rückgezogenen Unteransprüchen unter Schutz gestellt.

Dadurch, dass man Verfahren zur Abtrennung einer oder mehrerer am stärksten ungesättigten Verbindung(en) aus einer flüssigen Mischung bereitstellt, wobei die Mischung zusätzlich eine oder mehrere weniger stark ungesättigte organische Verbindung(en) und/oder eine oder mehrere gesättigte organische Verbindung(en) umfasst, wobei man

(i) einen sauren Kationenaustauscher mit Silberionen belädt,
(ii) den beladenen Kationenaustauscher mit der flüssigen Mischung mischt,
(iii) die Mischung im Batch-Verfahren bei einer Temperatur rührt, die unterhalb des Siedepunktes des verwendeten Lösungsmittels liegt,
(iv) den Überstand abtrennt, und
(v) die am stärksten ungesättigte(n) Verbindung(en) vom Kationenaustauscher ablöst,

wobei

➢ die flüssige Mischung Roh-Estergemische, prozessierte Öle, Estergemische, Fettsäuregemische und/oder Derivate von mehrfach ungesättigten Fettsäuren umfasst,
➢ man die am stärksten ungesättigte (n) Verbindung (en) vom Kationenaustauscher ablöst, indem durch Zugabe von Natriumnitrat das Silber von dem Ionentauscher abgelöst wird oder indem man das Silber durch Zugabe einer Säure oder Base ablöst,

kann man nämlich die erfindungsgemäße Aufgabe erstaunlich einfach lösen.

Im Unterschied zu den oben genannten Methoden wird in der vorliegenden Anmeldung neben den zur Trennung benötigten Silberionen eine Matrix verwendet (Ionenaustauscher), die einen wesentlichen Beitrag zur Selektivität, Reaktionszeit und Güte der Trennung beisteuert. So kann bei im Stand der Technik beschriebenen Verfahren auch beobachtet werden, das obwohl Silberionen vorliegen, keine Trennung der PUFAs erreicht wird.

Im Allgemeinen kann beobachtet werden, dass mehrfach ungesättigte Fettsäuren unterschiedlich stark an den Tauscher binden. So wird in einigen Fällen eine quantitative Ablösung schon in einem polaren Lösungsmittel mit oder ohne zusätzliches Erwärmen beobachtet. Dies ist erklärbar durch den unterschiedlichen Komplexierungsgrad der PUFA. So kann eine dreifach ungesättigte PUFA maximal 6 Komplexbindungen und eine sechsfach ungesättigte PUFA maximal 12 Komplexbindungen eingehen und damit die stärkere Bindungsspezies bilden.

Die Ablösung der ungesättigten Verbindung vom Kationenaustauscher kann erfindungsgemäß bei einer Temperatur von -20°C bis 80 °C erfolgen. Bevorzugt erfolgt die Ablösung der ungesättigten Verbindung bei einer Temperatur von - 20°C bis 40°C, ganz besonders bevorzugt bei -20°C bis 10°C.

Erfindungsgemäß aus Gemischen isolierbare Verbindungen umfassen u.a. Fettsäuren (gesättigt, ungesättigt oder hochungesättigt), isomere Fettsäuren (cis, trans, konjugiert, isoliert), Fettsäure-Derivate (z.B. Ether oder Ester, wie beispielsweise Methylester, Ethylester, Wächsester, Triglyceride, Diglyceride, Monoglyceride), Modifizierte Fettsäuren (beispielsweise Hydroxyfettsäuren, Oxofettsäuren (Keto-), Aminfettsäuren) auch als Derivate, ungesättigte Alkene, ungesättigte Alkohole, ungesättigte Ether, ungesättigte Ketone oder Aldehyde, Aromaten, Steroide oder Steroidester, Zucker (Monomere, Oligomere, ...), Tocopherole, Astaxanthin.

Erfindungsgemäß aus Gemischen mit anderen organischen Verbindungen isolierbare Fettsäuren umfassen dabei u.a.: Hexadecadiensäuren, Hexadecatriensäuren, Hexadecatetraensäuren, Linolsäure, γ-Linolensäure, α-Linolensäure, Stearidonsäure, Arachidonsäure, Eicosatriensäuren, Eicosatetraensäuren, Eicosapentaensäuren, Docosapentaensäuren, Docosahexaensäure, Tetracosadiensäuren, Octacosaoctaensäuren sowie deren Salze bzw. Ester, Ether oder andere Derivate dieser Fettsäuren.

Bevorzugt werden die Fettsäureethylester mit diesem Verfahren gereinigt.

Im Allgemeinen sind alle Fettsäuren der Kettenlänge von C14-C30 bzw. Derivate solcher Fettsäuren erfindungsgemäß aufreinigbar, die mehr als zwei Doppelbindungen aufweisen.

Eine Trennung von Verbindungen in Abhängigkeit von der Stellung der Doppelbindungen oder der Konfiguration der Doppelbindungen ist mit dem erfindungsgemäßen Verfahren möglich.

Grundsätzlich gelingen bei Verwendung vollversilberter Tauscher die besten Selektivitäten und werden die höchsten Beladungen der Tauscher mit PUFA erreicht.

Erfindungsgemäß können als Ausgangsgemische natürliche Öle, z.B. Fischöle, Pflanzenöle, mikrobiell hergestellte Öle, prozessierte Öle, Estergemische, freie Fettsäuregemische, ungesättigte Verbindungen und/oder Derivate solcher Verbindungen eingesetzt werden.

Fischöle enthalten zum Beispiel zwischen 10-20% DHA und mikrobiell hergestellte Öle bis zu 60% DHA. Demnach enthalten die Öle auch einen unterschiedlichen Anteil abzutrennender Fettsäuren. Daraus ergibt sich für den Fachmann die Schwierigkeit eine konkrete Reinheit der durch den Aufreinigungschritt erzielbaren reinen DHA. Im Allgemeinen macht es daher mehr Sinn einen Aufreinigungsfaktor zu definierten, um die Güte der Reinigung zu beurteilen. Der Aufreinigungsfaktor kann im einfachsten Fall der Quotient aus der Reinheit der Produktes und der Reinheit des Substrates sein. Die Reinheiten können beispielsweise aus einer gaschromatographischen Analyse ermittelt werden (Einheit: Area%).

$$R_F = R_{\text{Fettsäure im Produkt}}/R_{\text{Fettsäure im Substrat}} * 100\%,$$

wobei

$R_F$ = Reinigungsfaktor (%)

R= Reinheit

Die Reinigungsfaktoren liegen nach einer erfolgreichen Aufreinigung immer > 1. Abhängig vom Ausgangsgemisch können aber auch höhere Werte erzielt werden, bei einem Ausgangsgemisch, das die Zielfettsäure zu 0,5% aufweist und nach Reinigung eine Reinheit von 95% beispielsweise 190.

Im Beispiel der DHAEE kann sowohl aus einem 47% -igen DHAEE-Gemisch als auch aus einem 80%-igem DHAEE-Gemisch eine hochreine 95% DHAEE hergestellt werden. Die Reinigungsfaktoren liegen in diesen Beispielen zwischen 1,19 und 2,02.

Zur Durchführung des erfindungsgemäßen Verfahrens wird keine Chromatographiesäule benötigt und es muß keine Fraktionierung im eigentlichen Sinne durchgeführt werden.

Vor der Trennung von Überstand und Produktphase muss sich nur ein einziges Gleichgewicht einstellen. Dies steht ganz im Gegensatz zur Chromatographie (z.B. HPLC, SFC) bei der eine Trennung erst nach Erreichen einer bestimmten Bodenzahl (z.B. Säule), bzw. nach Einstellen einer u.U. sehr hohen Zahl an Gleichgewichten erreicht wird.

Technische Probleme, die normalerweise durch den Wechsel des Lösungsmittels in der Chromatographie auftreten (Luftblasen, Quellen des Tauschers, Inhomogenitäten) treten bei dem erfindungsgemäßen Verfahren nicht auf. Ebenfalls treten keine Flußprobleme (inhomogener Fluß) über die Trennsäule (Gradientenbildung) ein. Gleichzeitig können höhere Reinheiten des Produktes erreicht werden. Eine gezielte Einstellung der Produktreinheit ist ebenfalls möglich.

Überraschenderweise konnte von den vorstehend genannten Erfindern gezeigt werden, dass hochungesättigte Fettsäuren bzw. Derivate dieser hochungesättigten Fettsäuren (z.B. DHAEE) aus einer Lösung (z.B. DHAEE/DPAEE) im Batch-Verfahren hochselektiv an einen mit Silber-beladenen Kationentauscher in einem Schritt komplexiert werden können.

Die Selektivität der Komplexierung läßt sich dabei überraschenderweise sehr einfach in Abhängigkeit von der zu trennenden Fettsäurekonzentration, der Art des verwendeten Ionenaustauschers und dessen Menge, dem verwendeten Lösungsmittel, der Temperatur, der Zeit und der Silberbeladung des Ionenaustauschers steuern.

Diese Abhängigkeiten können für die Aufreinigung gezielt eingesetzt werden. Durch das Einstellen bestimmter Bedingungen lassen sich hochreine PUFAs ohne aufwendige Chromatographieschritte herstellen.

Bei dem neuen Verfahren handelt es sich daher nicht um ein Chromatographieverfahren, sondern um ein selektives Extraktionsverfahren (Komplexierung höherungesättigter Fettsäuren/Derivate an den oder die Kationentauscher und einen nachfolgenden Dekomplexierungsschritt in einem einfachen Rührgefäß im Batch).

Erfindungsgemäß einsetzbare Kationentauscher finden sich in der folgenden Liste unter Angabe der üblichen Handelsnamen: Dowex® 50 WX8, Dowex® 50 WX4, Dowex® 50 WX2, Dowex® MWC1, Dowex® MSC1, Dowex® Monosphere C-350, Dowex® CCR-2, Dowex® DR 2030, Amberlite® CG50, Amberlite® IR-120, Amberlyst® 15, Bio-Rex® 70 Resin, Macherey & Nagel PS-DVB®. Insbesondere sind solche Kationentauscher einsetzbar, die folgende Eigenschaften aufweisen:

Stark saure Kationenaustauscher: Gele, die als Grundkörper Styrol mit Divinylbenzolverzweigungen aufweisen, als aktive silbertragende Gruppe Sulfonsäure- und/oder Carboxylgruppen tragen und mikroporös oder bevorzugt macroporös sind. Insbesondere sind auch macroreticulare Ionenaustauscher besonders geeignet, da sie lösungsmittelstabil sind und im Vergleich zu Gelen eine wesentlich größere Oberfläche aufweisen. Diese tragen ebenfalls Sulfonsäure- und/oder Carboxylgruppen als funktionelle Gruppen.

Besonders bevorzugt einsetzbar sind Amberlyst® 15 und Dowex® DR2030.

Die Beladungskapazität der erfindungsgemäß einsetzbaren Kationentauscher reicht dabei von 0,1 bis 15 Gew.-% (g PUFA/100 g H$^+$-Tauscher) Gesamt-PUFA.

Dieses neue Verfahren stellt auf überraschend einfache Weise eine enorme Vereinfachung und ökonomische Verbesserung der bisher im Stand der Technik beschriebenen Aufreinigungsprozesse dar. Auch ist es ohne Probleme möglich, dieses Verfahren großtechnischen und industriellen Zwecken anzupassen, da beispielsweise Säulen etc. keine Verwendung finden.

Völlig überraschend war auch die Beobachtung, dass die Reinheit des Produktes am Kationentauscher durch nachfolgenden Waschvorgang mit einem Lösungsmittel und anschließender Erwärmung in einem bzw. mehreren Lösungsmitteln deutlich erhöht und selektiv gesteuert werden kann.

Die Wärmezufuhr kann erfindungsgemäß beispielsweise durch den Einsatz externer Wärmequellen, durch elektromagnetische Strahlung, wie beispielsweise Mikrowellen oder Infrarotstrahlung, oder aber durch Ultraschallbehandlung erfolgen. Jegliche dem Prozess förderliche Wärmequellen können Anwendung finden.

Es wird dabei vermutet, dass durch den Einfluss von Wärme (Energie) nicht vollständig komplexierte Fettsäure, bei der nicht alle Doppelbindungselektronen komplexiert vorliegen (dabei kann es sich um die Produktfettsäure, aber auch um Nebenfettsäuren oder Derivate handeln), dekomplexiert wird, wobei bevorzugt die höherungesättigte Fettsäure nach der Dekomplexierung wieder an den Kationentauscher komplexiert und die dort freigewordenen Positionen belegt. Die ebenfalls dekomplexierte Nebenfettsäure wird nach dieser Theorie somit nur in untergeordnetem Maße an den mit Silber beladenen Kationentauscher rückangelagert und verbleibt zum größten Teil im Überstand. Die Reinheit der Produktfettsäure am Kationentauscher wird dadurch beträchtlich erhöht.

Erfindungsgemäß kann daher dem System nach der Komplexierung der Fettsäure an den mit 100% Silber beladenen Kationentauscher Wärme zugeführt werden, um eine höhere Reinheit der Fettsäure zu gewährleisten. Bevorzugt wird dieser Schritt bei einer Temperatur T > 40°C und Druck P = 1 bar durchgeführt. Besonders bevorzugt wird der Schritt zwischen 40°C und 80°C durchgeführt. Ganz besonders bevorzugt zwischen 50 und 70°C und ganz besonders bevorzugt bei 55°C bis 65°C.

Wird bei Temperaturen von > 40°C gearbeitet, so kann die Dauer der Komplexierungsreaktion in 30 Minutenschritten inkrementiert sein, wobei ganz allgemein längere Reaktionszeiten eine höhere Produktreinheit ergeben. Allerdings hat sich gezeigt, dass 2,5 h in den meisten Fällen ausreichend sein dürften.

Auf den Einsatz von Acetonitril als Dekomplexierungsmittel kann im erfindungsgemäßen Verfahren bei PUFA oder PUFA-Derivaten mit weniger als 4 Doppelbindungen vollständig verzichtet werden, da Bedingungen erarbeitet wurden, bei denen

(i) die Ablösung in physiologisch unbedenklichen Lösungsmitteln gelingt und

(ii) ein völlig neuartiges Regenerationsverfahren für den Kationentauscher bzw. das Silber selbst entwickelt wurde.

Ein erfindungsgemäß einsetzbares Lösungsmittel für die Anlagerung der PUFA bzw. des Derivats kann aus den folgenden Gruppen ausgewählt sein: Alkane, Ketone, Ether, Ester, Diketone, Diester, Diether, Diole, Polyole, Nitrile, Dinitrile und Alkohole, besonders geeignete Ketone sind Aceton und 2-Methylethylketon, besonders geeignete Alkohole sind die mittelkettigen Alkohole 2-Propanol, Propanol, Butanol, Hexanol oder Isomere hierzu, noch mehr geeignet sind die Alkane n-Hexan, n-Heptan, n-Octan oder Isomere hierzu und am besten geeignet ist das Nitril Acetonitril, sowie die kurzkettigen Alkohole Ethanol und Methanol. Ethanol und Methanol sind die bevorzugten Lösungsmittel.

Erfindungsgemäß kann das Silber nach jedem Anlagerungsschritt von Fettsäuren an den Ionentauscher durch Zugabe einer Säure (z.B. $HNO_3$) oder Base (z.B. $NaHCO_3$) abgelöst werden. Damit wird gleichzeitig die komplexierte Fettsäure freigesetzt. Das Silber fällt als Salz aus (z.B. $Ag_2CO_3$) oder verbleibt in Lösung ($AgNO_3$) und kann durch Dekantieren bzw. Extraktion von der Produktfettsäure abgetrennt werden.

Anschließend kann das Silber erfindungsgemäß durch Zugabe einer Base (bei einer vorhergehenden Ablösung durch $HNO_3$ beispielsweise durch Zugabe von $NaHCO_3$) bzw. einer Säure (bei einer vorhergehenden Ablösung durch $NaHCO_3$ beispielsweise durch Zugabe von $HNO_3$) wieder in Lösung gebracht werden.

Dabei sind u.a. folgende Basen erfindungsgemäß einsetzbar: Hydroxide und Carbonate, organische Basen, insbesondere Natriumhydroxid, Kaliumhydroxid, bevorzugt Triethylamin, Calciumcarbonat, Magnesiumcarbonat, ganz besonders bevorzugt Kaliumcarbonat, Kaliumhydrogencarbonat, Natriumcarbonat und Natriumhydrogencarbonat.

Dabei sind u.a. folgende Säuren erfindungsgemäß einsetzbar: Phosphorsäure, insbesondere Salzsäure, Ameisensäure und Schwefelsäure, ganz besonders bevorzugt Essigsäure und Salpetersäure.

Durch die höhere Affinität der meisten Ionentauschermaterialien gegenüber $Ag^+$ im Vergleich zu anderen Kationen ($Ag^+ >> Na+ >> H^+$) gelingt die quantitative Anlagerung des Silbers an den Kationentauscher. Nach verschiedenen Waschschritten ist der Kationentauscher wieder einsetzbar. Die Wiederanlagerung des Silbers liegt dabei bei mindestens 90%, bevorzugt bei mindestens 95% und ganz besonders bevorzugt bei > 99% im Vergleich zur ersten Anlagerung.

Mit dem erfindungsgemäßen Verfahren können Reinigungsfaktoren von 1,01 bis 99 erzielt werden, wobei diese Reinigungsfaktoren zwar auch vom Verfahren, allerdings auch von der Konzentration der Zielverbindung in dem Ausgangsgemisch abhängen. Obwohl daher hohe Reinigungsfaktoren günstig sind, kann es dennoch von immenser Bedeutung sein, beispielsweise eine Zielfettsäure von 80% Reinheit auf 90% zu bringen, gerade wenn - wie in den Beispielen gezeigt - Moleküle voneinander getrennt werden, die sich nur im Vorhandensein einer einzigen weiteren Doppelbindung voneinander unterscheiden. Der entsprechende Reinigungsfaktor beträgt in diesem Beispiel zwar "nur" 1,125, in Verbindung mit den hohen erfindungsgemäßen Ausbeuten bedeutet dies jedoch eine dramatische Weiterentwicklung aller im Stand der Technik beschriebenen, z.B. chromatographischen Methoden und Verfahren.

Im erfindungsgemäßen Verfahren bei PUFA oder PUFA-Derivaten mit 4 oder mehr Doppelbindungen kann ein vollversilberter Kationentauscher verwendet werden, von dem die Ablösung der PUFA durch Regeneration des Kationentauschers bzw. des $Ag^+$, wie vorher beschrieben, gelingt.

Ein erfindungsgemäß einsetzbares Lösungsmittel für die Ablösung der PUFA bzw. des Derivats kann aus den folgenden Gruppen ausgewählt sein: Ketone (beispielsweise Aceton, 2-Methylethylketon), bevorzugt mittelkettige Alkohole (beispielsweise 2-Propanol, Butanol, Propanol), ganz besonders bevorzugt kurzkettige Alkohole (beispielsweise Methanol oder Ethanol) oder Gemische aus diesen Lösungsmitteln.

Die Erfindung bezieht sich auf ein Verfahren zur Auftrennung einer flüssigen Mischung nach dem Sättigungsgrad der in der Mischung enthaltenen Verbindungen. Der Sättigungsgrad richtet sich nach der Anzahl der Doppelbindungen. Die Mischung kann hochungesättigte organische Verbindungen, ungesättigte organische Verbindungen, aber auch gesättigte organische Verbindungen und auch anorganische Verbindungen enthalten.

**Die folgenden Figuren erläutern die Erfindung näher:**

Figur 1 zeigt das Prinzip der Aufreinigung von PUFAs durch selektive Extraktion vom $Ag^+$-Ionenaustauscher je nach verwendbarem Lösungsmittel

Figur 2 zeigt ein Schema der erfindungsgemäßen Komplexierung von Fettsäureethylestern am $Ag^+$-Ionentauscher

Figur 3 zeigt ein Schema der erfindungsgemäßen Regeneration des Silbers

**Verwendete Abkürzungen:**

DHA      All-cis-4,7,10,13,16,19-Docosahexaensäure

DPA      All-cis-4,7,10,13,16-Docosapentaensäure ($\omega$-6)

DHAEE     All-cis-4,7,10,13,16,19-Docosahexaensäureethylester
DPAEE     All-cis-4,7,10,13,16-Docosapentaensäureethylester ($\omega$-6)
SFC       Supercritical fluid chromatography
PUFA      Polyunsaturated fatty acid

**Verwendete Begriffe:**

**Beladungskapazität**

Die Beladungskapazität des Kationenaustauschers beschreibt die Menge PUFA oder definiertes Derivat (z.B. Docosahexaensäureethylester; DHAEE) in g oder Prozent (%, w/w), die an 100 g des in der protonierten Form ($H^+$-Form) befindlichen Kationenaustauschers komplexiert werden kann.

Die Erfindung wird anhand der nachfolgenden Beispiele veranschaulicht, ohne dass diese als Einschränkung der Erfindung verstanden werden dürfen.

Wenn nicht anders angegeben, wurde in allen nachfolgenden Beispielen als Substrat für die Aufreinigung ein Gemisch von etwa 80% DHAEE und 20% DPAEE ($\omega$-6) verwendet, das aus einer mikrobiellen Fermentation mit dem Stamm *Schizochytrium* SR21 gewonnen werden kann [Yokochi et al., Optimization of docosahexaenoic acid production by Schizochytrium limacinum SR21", Appl. Microbiol. Biotechnol. (1998), 49: 72-76]. Das aus dieser Fermentation erhaltene Öl aus *Schizochytrium* wurde in die Ethylester umgeestert und einer Fällung mit Harnstoff unterzogen, wobei die gesättigten Fettsäuren entfernt werden. DHAEE und DPAEE können weiter mittels HPLC aufgereinigt werden [Yamamura, supra], und als Substrat für die Aufreinigunsversuche eingesetzt werden. Die Trennung der Ethylester voneinander in quantitativ bedeutenden Mengen stellt ein großes Problem dar, da sich die beiden Fettsäuren lediglich in der unterschiedlichen Anzahl der Doppelbindungen unterscheiden.

**Beispiel 1**

**Herstellung von zu 100% mit Silberionen beladenem Amberlyst®15 (20-50 mesh)**

Die Herstellung der silberbeladenenen Ionenaustauscher erfolgt in Anlehnung an das von Nieto *et al.* (Nieto, S.; A. M. Cordoba; J. Sanhuenzy and A. Valenzuela (1997): Obtention of highly purified fractions of eicosapentaenoic acid and docosahexaenoic acid from sardine oil by silver-resin chromatography: A semi-preparative procedure. Grasas y Aceites, 48(4), 197-199) für Dowex® 50WX8 (frühere Bezeichnung Dowex® W-HCR-W2) beschriebene Verfahren. Dazu wurde das Verfahren jedoch deutlich vereinfacht bzw. abgewandelt.

Es erfolgt weder eine Vorbereitung des Materials in einer beheizbaren Gassäule noch wird eine Vorwaschung des Materials mit organischen Lösungsmitteln durchgeführt. In dem erfindungsgemäßen neuen Verfahren ist jedoch im Gegensatz zu dem von Nieto *et al.* offenbarten Verfahren die Korngröße des Materials ausschlaggebend für die Qualität der Trennung und die Ausbeute sein.

20 g Amberlyst® 15 werden in eine Nutsche oder Glassäule mit Nutsche gegeben und mit 1 M Natriumnitrat-Lösung ($NaNO_3$) solange gespült, bis der pH-Wert des Eluates von sauer nach neutral umschlägt. Die Neutralisation zeigt die verminderte Bildung von Salpetersäure an, die beim Austausch von Protonen zu Natrium-Ionen gebildet wird. Ist der Kationentauscher vollständig mit Natrium-Ionen beladen bleibt der Eluent neutral.

Jetzt kann auf zwei unterschiedliche Weisen weiter verfahren werden.

Entweder wird der Natrium-beladene Kationentauscher solange mit 0,4 M Silbernitrat-Lösung gespült, bis im Eluenten Silber nachweisbar ist, oder das Material wird zuerst in einen Rundkolben oder Erlenmeyerkolben mit Natriumnitrat-Lösung überspült. Die überschüssige Natriumnitrat-Lösung wird dann verworfen.

Im nachfolgenden wird mit 5,4 ml 0,4 M Silbernitrat-Lösung/g Amberlyst® 15 für 8-12 h gerührt. Der Überstand wird entfernt.

In beiden Fällen verfährt man mit dem $Ag^+$-beladenen Kationentauscher folgendermaßen:

Der mit Silber beladene Kationentauscher (ca. 2,0 mmol $Ag^+$/ml $H^+$-Tauscher, wobei 1 g $H^+$-Dowex ca. 0,9 ml $H^+$-Dowex entspricht) wird dreimal mit 100 ml Wasser silberfrei gewaschen und anschließend zweimal mit 100 ml Ethanol wasserfrei gewaschen (1 h). Nachfolgend läßt man in 100 ml Acetonitril über Nacht (12h) stehen. Danach wird erneut zweimal mit jeweils 100 ml Ethanol gewaschen. Das Material kann jetzt verwendet werden. Acetonitril kann auch durch dreimaliges Verwenden von 100 ml Ethanol ersetzt werden.

Ein anderer Waschvorgang bzw. Aktivierungsvorgang kann zu vermindert aktivem oder nicht aktivem mit Silber beladene Kationentauscher führen. Dabei ist der Gehalt an Wasser im mit Silber beladenen Kationentauscher von besonderer Bedeutung.

**Beispiel 2**

**Herstellung des zu 50% partiell mit Silberionen beladenen Amberlyst® 15 (20-50mesh) Ionenaustauschers**

Teilversilbertes Amberlyst®-Material wird unter Rühren hergestellt, um eine gleichmäßige Verteilung des Silbers am Kationentauscher zu ermöglichen.

50% versilbertes Material Amberlyst® 15 wird durch Zugabe von 2,7 ml 0,4 M Silbernitrat-Lösung/g Amberlyst® 15 für 8-12 h hergestellt. Dabei wird der Ansatz 12 h gerührt werden. Danach wird der Überstand dekantiert und der mit Silber beladene Kationentauscher dreimal mit bidestilliertemWasser gewaschen. Anschließend erfolgt zweifaches Waschen mit 100 ml Ethanol (jeweils 1h) und einfaches Waschen mit 100 ml Acetonitril (12 h). Das Acetonitril kann durch mehrfache Verwendung von Ethanol ersetzt werden. Das Material wird letztendlich in Ethanol aufgenommen und danach eingesetzt.

In gleicher Weise kann Material von *0,1-99,9*% (bezogen auf die 100% Versilberung) Versilberung hergestellt werden.

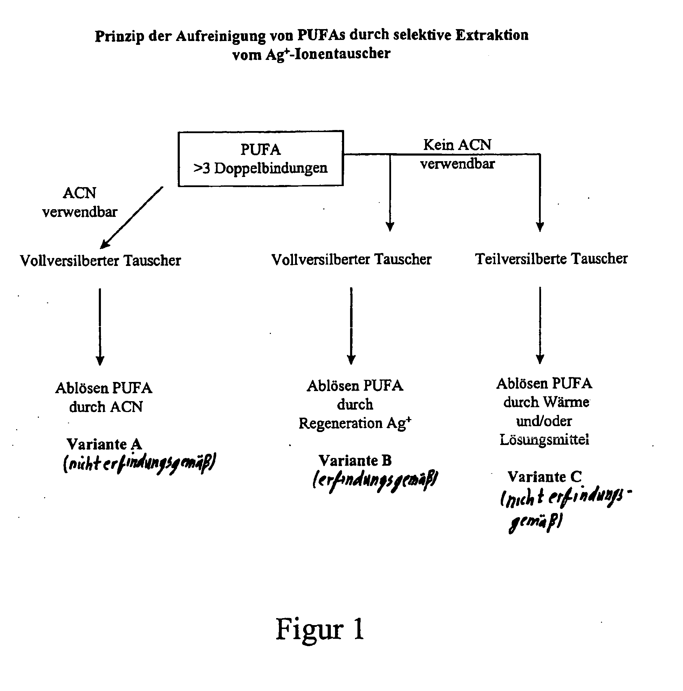

**Verschiedene Ablöseprotokolle (Abb. 1)**

**Variante A. Ablösen der Produkte vom Ag⁺-Tauscher mit einem π-Elektronendonor (nicht erfindungsgemäß)**

In Abhängigkeit vom Tauschermaterial, Silberbeladung und von dem angelagerten Fettsäureethylester (Anzahl der Doppelbindungen) ist das Lösungsmittel auszuwählen, das den Produktester vom mit Silber beladenen Kationentauscher ablösen kann. Im idealen Fall kann ein Lösungsmittel verwendet werden, das ebenfalls als π-Elektronendonor dient. Beispiele sind hierfür Acetonitril, 1-Hexen oder Toluol.

Z. B. kann gebundener DHAEE in einfachster Weise durch Zugabe von Acetonitril (ACN) vom mit Silber beladenen Kationentauscher abgetrennt werden. Das Acetonitril stellt in diesem Fall den stärkeren π-Elektronendonor als die PUFAs dar.

Dazu werden dem Ansatz 100 ml ACN zugegeben und es wird bei Raumtemperatur unter Schutzgas gerührt. Nach vollständiger Ablösung des Fettsäureethylesters wird die organische Phase abgenommen. Das Tauschermaterial wird mit ACN nachgewaschen und die organischen Phasen vereint. Nach Abziehen des Lösungsmittels erhält man den aufgereinigten DHAEE als schwach gelbes Öl. In allen aufgeführten Beispielen reichen hierfür schon geringere Mengen an Acetonitril, beispielsweise ein Gemisch von Acetonitril in n-Hexan (10:90, v:v).

Die folgenden Beispiele 3-9 stellen Beispiele für solche Verfahren dar, bei denen ACN zum Ablösen der komplexierten Fettsäure vom Tauscher verwendet wird.

**Beispiel 3**

**Anlagerungsversuch eines Gemisches von DHAEE und DPAEE an Ag⁺-Amberlyst® 15 (20-50mesh) in Ethanol über 24 Stunden:**

In Vorversuchen wurde ermittelt, dass die PUFA-Beladungskapazität des Tauschers bei ungefähr 5 Gew.-% PUFA liegt (g PUFA/100 g H⁺-Tauscher).

597,8 mg Docosahexaensäureethylester (DHAEE) und 144,0 mg Docosapentaensäureethylester (ω-6 DPAEE) wurden zu einer gerührten Lösung des mit 100% Silber-beladenen Tauschers (10 g H⁺-Amberlyst® 15) aus Beispiel 1 in 100 ml absolutem Ethanol gegeben. Die Suspension wird bei Raumtemperatur unter Schutzgas für 24 h bei RT bei 100 U/min geschüttelt. Gegebenenfalls kann noch ein Antioxidant zugegeben werden (z.B. Tocopherol, 2,6-Di-tert.-butyl-4-methylphenol (BHT), Ascorbylpalmitat), um Oxidationen der PUFAs zu vermeiden.

Nach jeweils 30 oder 60 min Zeitabständen wird die Reaktion quantitativ und qualitativ erfaßt, indem der Überstand gaschromatographisch analysiert wird (HP GC6890, Säule: Macherey & Nagel FFAP Permabond 0,1 μm (25m, 0,25mm), Splitbetrieb (10:1), Trägergas: Helium (constant flow 1,0 ml/min), FID-Betrieb mit Wasserstoff (30 ml/min) und Sauerstoff (300 ml/min) als Brenngase, Make up: 20 ml Helium, Detektor- und Injektortemperatur: jeweils 255° C, GC-Ofen-Temperaturprogramm: Anfangstemperatur 180° C, Temperaturanstiegsrate 10° C/min auf Endtemperatur 230° C, diese für 5min halten, Injektionsvolumen: 1,0 μl). Durch Zugabe eines internen Standards zum Reaktionsansatz, der nicht an den mit Silber beladenen Kationenaustauscher bindet (z.B. eine gesättigte Fettsäure bzw. ein Ester einer gesättigten Fettsäure oder ein anderes Derivat) kann eine quantitative Analyse der hochungesättigten Fettsäuren im Überstand durchgeführt werden.

Dabei kann die theoretische Beladung des Tauschers durch Subtraktion des im Überstand verbliebenen Fettsäureethylesters von der insgesamt eingesetzten Fettsäureethylester berechnet werden. Die Reinheit des Produktes kann ebenfalls so ermittelt werden (Tab. 1).

In diesem Fall wurde fast der gesamte DHAEE (86,7%) am Tauscher komplexiert. Die Reinheit des DHAEE wurde von 80% auf fast 91 % gesteigert. Die Komplexierung ist bereits nach wenigen Stunden beendet.

Ist der Ionenaustauscher vollständig mit PUFAs beladen (keine weitere Anlagerung mehr beobachtbar) oder die gewünschte Reinheit (z.B. DHAEE > 90%) erreicht, wird der Überstand abgenommen, und das Ionentauschermaterial wird einmal mit 100 ml Ethanol gewaschen und von Resten nicht-gebundenem Fettsäureethylesters befreit. Nach beendeter Reaktion kann der gebundene Produktfettsäureethylester durch verschiedene Verfahren vom mit Silber beladenen Kationentauscher abgelöst werden:

Am einfachsten kann DHAEE durch Ausrühren mit 100 ml Acetonitril für 7 h unter Schutzgas abgelöst werden (nicht erfindungsgemäß). Dazu reicht schon eine 10%-ige Mischung von Acetonitril in Ethanol oder n-Hexan. Nach Entfernen der Lösungsmittel wurden 482.6 mg des aufgereinigten DHAEE in einer Reinheit von 88,4% isoliert.

Dieser Wert stimmt mit dem berechneten Wert aus Tabelle 1 gut überein. Die Ausbeute an DHAEE beträgt 81%. Die Produktbeladung des Tauscher beträgt somit 4,8% (g DHAEE/100 g $H^+$-Tauscher).

Aus dem Überstand konnten nach Entfernen der Lösungsmittel weitere 66,4 mg DHAEE isoliert werden. Die Gesamtwiederfindung DHAEE betrug 92%.

**Tab.1: Ergebnisse der Anlagerung eines Gemisches von DHAEE und DPAEE an Ag⁺-Amberlyst® 15**

| Zeit | Ü DPAEE | Ü DHAEE | Ü DPAEE | Ü DHAEE | Summe | WF in EtOH | DHAEE Amberlyst | DPAEE Amberlyst | DHAEE Amberlyst | Ausbeute DHAEE Amberlyst | Reinigungsfaktor DHAEE |
|---|---|---|---|---|---|---|---|---|---|---|---|
| (h) | (Area-%) | (Area-%) | (mg) | (mg) | (mg) | (%) | (mg) | (mg) | (Area-%) | (%) | |
| 0 | 20,5 | 79,5 | 146,6 | 567,7 | 714,3 | 96,3 | 30,1 | -2,5 | 109,2 | 5,0 | |
| 1 | 39,0 | 61,0 | 100,7 | 158,3 | 259,0 | 34,9 | 439,4 | 43,3 | 91,0 | 73,5 | 1,14 |
| 2 | 45,0 | 55,0 | 99,0 | 121,4 | 220,4 | 29,7 | 476,4 | 45,0 | 91,4 | 79,7 | 1,14 |
| 3 | 46,4 | 53,6 | 100,8 | 116,6 | 217,4 | 29,3 | 481,2 | 43,3 | 91,8 | 80,5 | 1,15 |
| 7 | 50,8 | 49,2 | 96,8 | 94,0 | 190,8 | 25,7 | 503,8 | 47,2 | 91,4 | 84,3 | 1,14 |
| 24 | 53,6 | 46,4 | 91,8 | 79,4 | 171,2 | 23,1 | 518,4 | 52,2 | 90,8 | 86,7 | 1,14 |

Ü: Überstand, WF=Wiederfindung, Amberlyst® 15

**Beispiel 4**

**Anlagerungsversuch eines Gemisches von DHAEE und DPAEE an Ag⁺-Amberlyst® 15 (20-50 mesh) in Ethanol über 210 Minuten**

Dazu wird eine Mischung aus 0,585 g Docosahexaensäureethylester (DHAEE) und 0,146 g Docosapentaen-säureethylester ($\omega$-6 DPAEE) zu einer gerührten Lösung des mit 100% Silber-beladenen Tauschers (entspricht 10 g H⁺-Amberlyst®15) in 100 ml absolutem Ethanol gegeben. Die Suspension wird bei 26 °C unter Schutzgas für 210 min bei 100 U/min geschüttelt. Zu den angegebenen Zeiten wurde der Überstand gaschroamtographisch analysiert.

Die Reinheit und Ausbeute des Produktes kann ebenfalls so ermittelt werden (Tab. 2).

Im Vergleich zum Anlagerungsversuch mit Dowex®50WX8 200-400 mesh (Beispiel 6, Tabelle 4) zeigt sich eine erheblich reduzierte Reaktionszeit und eine deutliche Zunahme der Beladung mit DHAEE von 1,2% (g DHAEE/100g H⁺-Dowex50WX8) auf 4,6% (g DHAEE/100g H⁺-Amberlyst®). Die Reinheit des DHAEE konnte von 80% auf über 92,9% gesteigert werden. Die Ausbeute an 92,9%-igem DHAEE beträgt 77,9%. Der vergleichbare Tauscher (Korngröße) aus der Dowex®50WX8 Serie (20-40 mesh) zeigt keine DHAEE-Beladungskapazität und damit auch keinen Reinigungseffekt.

**Tab. 2: Ergebnisse der Anlagerung eines Gemisches von DHAEE und DPAEE an Ag⁺-Amberlyst® 15 (20-50 mesh)**

| Zeit | Ü DPAEE | Ü DHAEE | Ü DPAEE | Ü DHAEE | Summe | WF in EtOH | Amberlyst DHAEE | Amberlyst DPAEE | Amberlyst DHAEE | Ausbeute DHAEE | Reinigungsfaktor DHAEE |
|------|---------|---------|---------|---------|-------|------------|-----------------|-----------------|-----------------|----------------|------------------------|
| (min) | (Area-%) | (Area-%) | (mg) | (mg) | (mg) | (%) | (mg) | (mg) | (Area-%) | (%) | |
| 0 | 20,6 | 79,4 | 180,9 | 696,4 | 877,3 | 120,0 | -111,4 | -34,9 | 76,1 | -19,0 | |
| 10 | 25,6 | 74,4 | 130,7 | 380,2 | 510,9 | 69,9 | 205,0 | 15,3 | 93,0 | 35,0 | 1,16 |
| 20 | 29,1 | 70,9 | 119,2 | 290,3 | 409,5 | 56,0 | 294,8 | 26,8 | 91,7 | 50,4 | 1,15 |
| 30 | 32,3 | 67,7 | 118,8 | 248,8 | 367,5 | 50,3 | 336,3 | 27,2 | 92,5 | 57,5 | 1,16 |
| 45 | 35,5 | 64,5 | 117,1 | 212,2 | 329,3 | 45,0 | 372,9 | 28,9 | 92,8 | 63,7 | 1,16 |
| 60 | 37,3 | 61,2 | 117,3 | 192,5 | 309,7 | 42,4 | 392,7 | 28,7 | 93,2 | 67,1 | 1,17 |
| 150 | 44,2 | 55,8 | 114,4 | 144,5 | 258,9 | 35,4 | 440,6 | 31,6 | 93,3 | 75,3 | 1,17 |
| 210 | 43,9 | 51,0 | 111,4 | 129,5 | 240,8 | 32,9 | 455,7 | 34,7 | 92,9 | 77,9 | 1,16 |
| Ü: Überstand, T: mit Silber beladener Kationentauscher, WF=Wiederfindung | | | | | | | | | | | |

EP 1 392 621 B1

**Beispiel 5**

**Anlagerungsversuch eines Gemisches von DHAEE und DPAEE an Ag⁺-Amberlyst® 15 (20-50mesh) in n-Hexan:**

Dazu werden 585,1 mg Docosahexaensäureethylester (DHAEE) und 146,0 mg Docosapentaensäureethylester ($\omega$-6 DPAEE) zu einer gerührten Lösung des vollständig mit Silber beladenen Tauschers (9,3 g H⁺-Amberlyst® 15) in 100 ml n-Hexan gegeben. Die Suspension wird bei Raumtemperatur unter Schutzgas für 3,5 h bei 100 U/min geschüttelt.

Nach jeweils 30 oder 60 min Zeitabständen wird die Reaktion quantitativ und qualitativ erfaßt, indem der Überstand gaschromatographisch analysiert wird. Dabei kann die theoretische Beladung des Tauschers durch Subtraktion berechnet werden. Die Reinheit des Produktes kann ebenfalls so ermittelt werden (Tab. 3).

Die Reinheit der DHAEE wurde 80% auf über 94,5% gesteigert. Die Anlagerung ist bereits nach wenigen Minuten praktisch beendet. Die Beladung des Tauschers mit DHAEE beträgt 4,4% DHAEE/100g H⁺-Tauscher). Die Ausbeute an 94,5%-igem DHAEE beträgt 74,6 %.

Der Überstand wird abgenommen, das Kationenaustauschermaterial einmal mit 100 ml n-Hexan gewaschen und von Resten nicht-gebundenem Fettsäureethylesters befreit. Nach beendeter Reaktion kann der gebundene Produktfettsäureethylester durch verschiedene Verfahren von dem mit Silber beladenen Kationentauscher abgelöst werden.

In einfachster Weise kann DHAEE durch Ausrühren mit 100 ml Acetonitril für 7 h unter Schutzgas abgelöst werden. Anstelle des reinen Acetonitrils kann aber auch schon eine 10%-ige Mischung von Acetonitril in n-Hexan eingesetzt werden, wodurch ein identisches Ergebnis erreicht wird (nicht erfindungsgemäß).

**Tab. 3: Ergebnisse der Anlagerung eines Gemisches von DHAEE und DPAEE an Ag$^+$-Amberlyst® 15**

| Zeit | Ü DPAEE | Ü DHAEE | Ü DPAEE | Ü DHAEE | Summe | WF in EtOH | DHAEE Amberlyst | DPAEE Amberlyst | DHAEE Amberlyst | Ausbeute DHAEE | Reinigungsfaktor DHAEE |
|---|---|---|---|---|---|---|---|---|---|---|---|
| (min) | (Area-%) | (Area-%) | (mg) | (mg) | (mg) | (%) | (mg) | (mg) | (Area-%) | (%) | |
| 0 | 20,7 | 79,3 | 158,8 | 609,1 | 767,9 | 105,0 | -24,0 | -12,7 | 65,3 | -4,1 | |
| 10 | 29,0 | 71,0 | 118,2 | 290,1 | 408,3 | 55,8 | 295,1 | 27,9 | 91,4 | 50,4 | 1,14 |
| 20 | 32,9 | 67,1 | 127,5 | 260,5 | 388,0 | 53,1 | 324,6 | 18,5 | 94,6 | 55,5 | 1,18 |
| 45 | 37,6 | 62,4 | 121,7 | 202,3 | 324 | 44,3 | 382,9 | 24,3 | 94,0 | 65,4 | 1,18 |
| 60 | 39,1 | 60,9 | 123,4 | 192,5 | 315,9 | 43,2 | 392,7 | 22,6 | 94,6 | 67,1 | 1,18 |
| 150 | 43,2 | 56,8 | 121,9 | 160,4 | 282,3 | 38,6 | 424,8 | 24,1 | 94,6 | 72,6 | 1,18 |
| 210 | 44,9 | 55,1 | 120,7 | 148,4 | 269,1 | 36,8 | 436,8 | 25,3 | 94,5 | 74,6 | 1,18 |
| Ü: Überstand, WF=Wiederfindung, Amberlyst: Amberlyst® 15 | | | | | | | | | | | |

**Beispiel 6**

**Anlagerungsversuch eines Gemisches von DHAEE und DPAEE an Ag$^+$-Amberlyst® 15 in n-Hexan bei erhöhter Temperatur:**

Dazu werden 1,18 g Docosahexaensäureethylester (DHAEE) und 0,29 g Docosapentaensäureethylester ($\omega$-6 DPAEE) zu einer gerührten Lösung des vollständig mit Silber-beladenen Tauschers (9,3 g H$^+$-Amberlyst®) in 100 ml n-Hexan gegeben. Die Suspension wird bei Raumtemperatur unter Schutzgas für 2 h bei 55 °C bei 100 U/min geschüttelt. Gegebenenfalls kann noch ein Antioxidant zugegeben werden (z.B. Tocopherol, Ascorbylpalmitat), um Oxidationen der PUFAs zu vermeiden.

Nach jeweils 30 oder 60 min Zeitabständen wird die Reaktion quantitativ und qualitativ erfaßt, indem der Überstand gaschromatographisch analysiert wird. Dabei kann die theoretische Beladung des Tauschers durch Subtraktion berechnet werden. Die Reinheit des Produktes kann ebenfalls so ermittelt werden (Tab. 4).

Die Reinheit des DHAEE wurde von 79% auf 97,9% gesteigert. Die Anlagerung ist bereits nach wenigen Minuten beendet. Die Beladung des Tauschers mit DHAEE beträgt 5,5% (g DHAEE/100g H$^+$-Tauscher). Die DHAEE-Ausbeute liegt bei 47,1%.

**Tab. 4: Ergebnisse der Anlagerung eines Gemisches von DHAEE und DPAEE an Ag⁺-Amberlyst® 15 bei 55 °C**

| Zeit | Ü DPAEE | Ü DHAEE | Ü DPAEE | Ü DHAEE | Summe | WF in n-Hexan | DHAEE Amberlyst | DPAEE Amberlyst | DHAEE Amberlyst | Ausbeute DHAEE | Reinigungsfaktor DHAEE |
|------|---------|---------|---------|---------|-------|---------------|-----------------|-----------------|-----------------|----------------|------------------------|
| (min) | (Area-%) | (Area-%) | (mg) | (mg) | (mg) | (%) | (mg) | (mg) | (Area-%) | (%) | |
| 0 | 20,5 | 78,9 | 312,7 | 1205,6 | 1518,3 | 103,0 | -25,9 | -18,4 | 58,6 | -2,2 | |
| 10 | 25,8 | 73,5 | 300,2 | 855,7 | 1155,9 | 78,4 | 324,1 | -5,8 | 101,8 | 27,5 | 1,27 |
| 20 | 27,1 | 72,9 | 288,0 | 775,2 | 1063,2 | 72,1 | 404,5 | 6,4 | 98,4 | 34,3 | 1,23 |
| 30 | 27,8 | 72,2 | 283,5 | 736,7 | 1020,2 | 69,2 | 443,0 | 10,9 | 97,6 | 37,6 | 1,22 |
| 45 | 28,6 | 71,4 | 292,9 | 731,6 | 1024,4 | 69,5 | 448,2 | 1,5 | 99,7 | 38,0 | 1,25 |
| 60 | 29,5 | 70,5 | 290,5 | 693,7 | 984,2 | 66,8 | 486,0 | 3,9 | 99,2 | 41,2 | 1,24 |
| 120 | 30,9 | 68,3 | 282,6 | 623,9 | 906,5 | 61,5 | 555,8 | 11,8 | 97,9 | 47,1 | 1,22 |

Ü: Überstand, WF=Wiederfindung, Amberlyst: Amberlyst® 15

**Beispiel 7**

**Anlagerungsversuch eines Gemisches von DHAEE und DPAEE an Ag+-Dowex® 50WX8 (200-400) mesh:**

Es wurde weiterhin untersucht, welchen Einfluss die mesh-Größe des verwendeten Tauschermaterial auf die Trennung hat.

Dazu werden 291,4 mg Docosahexaensäureethylester (DHAEE) und 72,7 mg Docosapentaensäureethylester (ω-6 DPAEE) zu einer gerührten Lösung des vollständig mit Silber beladenen Tauschers (21 g H+-Dowex® 50WX8, 200-400 mesh) in 100 ml absolutem Ethanol gegeben. Die Suspension wird bei Raumtemperatur unter Schutzgas für 24 h bei 300 U/min mit einem Magnetrührer gerührt

Nach jeweils 30 oder 60 min Zeitabständen wird die Reaktion quantitativ und qualitativ erfaßt, indem der Überstand gaschromatographisch analysiert wird. Dabei kann die theoretische Beladung des Tauschers durch Subtraktion berechnet werden. Die Reinheit des Produktes kann ebenfalls so ermittelt werden (Tab. 5).

Die Reinheit der DHAEE wurde von 80% auf 91,7% gesteigert. Die Anlagerung ist nach 46 Stunden beendet. Die Beladung des Tauschers mit DHAEE beträgt 1,2% (g DHAEE/100 g H+-Tauscher). Die Ausbeute des 91,7%-igen DHAEE liegt bei 83,7%

Im Gegensatz dazu liegen die DHAEE-Beladungen bei dem vollversilbertem Dowex® 50WX8 100-200 mesh Material bei 1,1% (g DHAEE/100 g H+-Tauscher) und bei dem vollversilbertem Dowex® 50WX8 20-40 mesh Material praktisch bei 0% DHAEE/H+-Tauscher. Hier zeigt sich also eine ausgeprägte Abhängigkeit von der Korngröße des Tauschermaterials.

**Tab. 5: Ergebnisse der Anlagerung eines Gemisches von DHAEE und DPAEE an Ag+-Dowex® 50 WX8 (200-400 mesh)**

| Zeit: | Ü DPAEE | Ü DHAEE | Ü DPAEE | Ü DHAEE | Summe | WF in EtOH | DHAEE Dowex | DPAEE Dowex | DHAEE Dowex | Ausbeute DHAEE | Reinigungsfaktor DHAEE |
|---|---|---|---|---|---|---|---|---|---|---|---|
| 0 | (Area-%) | (Area-%) | (mg) | (mg) | (mg) | (%) | (mg) | (mg) | (Area-%) | (%) | |
| (h) | 17,7 | 73,2 | 66,9 | 277,6 | 344,5 | 94,6 | 13,8 | 5,7 | 70,6 | 4,8 | |
| 1 | 19,6 | 68,8 | 65,9 | 229,0 | 294,8 | 81,0 | 62,4 | 6,8 | 90,1 | 21,4 | 1,13 |
| 2 | 21,1 | 68,5 | 66,9 | 216,0 | 283,0 | 77,7 | 75,4 | 5,7 | 92,9 | 25,9 | 1,16 |
| 3 | 21,7 | 66,0 | 65,9 | 199,8 | 265,7 | 73,0 | 91,6 | 6,8 | 93,1 | 31,4 | 1,16 |
| 5 | 23,3 | 63,7 | 63,7 | 175,0 | 238,7 | 65,5 | 116,4 | 9,0 | 92,8 | 40,0 | 1,16 |
| 6,25 | 24,2 | 62,3 | 63,7 | 163,1 | 226,8 | 62,3 | 128,3 | 9,0 | 93,4 | 44,0 | 1,17 |
| 7 | 24,7 | 61,6 | 63,7 | 157,7 | 221,4 | 60,8 | 133,7 | 9,0 | 93,7 | 45,9 | 1,17 |
| 22 | 31,7 | 50,4 | 57,2 | 91,9 | 149,0 | 40,9 | 199,6 | 15,4 | 92,8 | 68,5 | 1,16 |
| 28 | 34,7 | 46,8 | 55,1 | 74,5 | 129,6 | 35,6 | 216,9 | 17,5 | 92,5 | 74,4 | 1,16 |
| 46 | 41,1 | 38,7 | 50,8 | 47,5 | 98,3 | 27,0 | 243,9 | 21,9 | 91,7 | 83,7 | 1,15 |
| Ü: Überstand, WF: Wiederfindung, Dowex: Dowex® WX8 | | | | | | | | | | | |

**Beispiel 8**

**Anlagerungsversuch eines Gemisches von DHAEE und DPAEE an Ag⁺-Dowex® 50WX8 mit nachfolgender Aufkonzentrierung durch Temperaturshift:**

Dazu werden 116,7 mg Docosahexaensäureethylester (DHAEE) und 29,2 mg Docosapentaensäureethylester ($\omega$-6 DPAEE) zu einer gerührten Lösung des vollständig mit Silber beladenen Tauschers (25 g H⁺-Dowex® 50WX8, 100-200 mesh) in 200 ml absolutem Ethanol gegeben. Die Suspension wird bei Raumtemperatur unter Schutzgas für 24 h bei 300 U/min mit einem Magnetrührer gerührt. Gegebenenfalls kann noch ein Antioxidant zugegeben werden (z.B. Tocopherol, Ascorbylpalmitat), um Oxidationen der PUFAs zu vermeiden.

Nach jeweils 30 oder 60 min Zeitabständen wird die Reaktion quantitativ und qualitativ erfaßt, indem der Überstand gaschromatographisch analysiert wird. Dabei kann die theoretische Beladung des Tauschers durch Subtraktion berechnet werden. Die Reinheit des Produktes kann ebenfalls so ermittelt werden (Tab. 6).

Die Reinheit des DHAEE wurde von 80% auf über 92,8% gesteigert. Die Anlagerung ist nach 24 Stunden beendet. Die Beladung des Tauschers mit DHAEE beträgt 0,40% (g DHAEE/ 100 g ⁺H-Tauscher).

Ein weitere Erhöhung der Reinheit der DHAEE am mit Silber beladenen Kationentauscher erhält man, indem man den mit PUFA beladenen Kationentauscher mit 100 ml Lösungsmittel (z.B. Ethanol) wäscht und anschließend in einem Lösungsmittel (z.B. Ethanol, Aceton) erwärmt. Der verwendete Temperaturbereich liegt dabei zwischen 50 °C und 80 °C (Tab. 7).

**Tab. 6: Ergebnisse der Anlagerung eines Gemisches von DHAEE und DPAEE an Ag⁺-Dowex® 50 WX8**

| Zeit (h) | Ü DPAEE (Area-%) | Ü DHAEE (Area-%) | Ü DPAEE (mg) | Ü DHAEE (mg) | Summe (mg) | WF in EtOH (%) | DHAEE Dowex (mg) | DPAEE Dowex (mg) | DHAEE Dowex (Area-%) | Ausbeute DHAEE (%) | Reinigungsfaktor DHAEE |
|---|---|---|---|---|---|---|---|---|---|---|---|
| 0 | 19,3 | 77,8 | 30,2 | 118,8 | 149,0 | 101,5 | -2,1 | -0,2 | 89,6 | -1,7 | |
| 1 | 21,8 | 74,0 | 28,1 | 95 | 123,1 | 84,2 | 21,7 | 1,3 | 94,5 | 18,6 | 1,18 |
| 2 | 24,1 | 72,4 | 28,1 | 82,1 | 101,2 | 74,9 | 34,7 | - 1,9 | 94,7 | 29,7 | 1,18 |
| 3,5 | 26,1 | 70,0 | 25,9 | 71,3 | 97,2 | 66,6 | 45,5 | :3,2 | 93,4 | 39,0 | 1,17 |
| 4,5 | 27,3 | 68,6 | 25,9 | 64,8 | 90,7 | 62,0 | 55,0 | 3,5 | 93,8 | 44,5 | 1,17 |
| 5,5 | 27,5 | 64,3 | 25,9 | 60,5 | 86,4 | 59,2 | 56,3 | 3,2 | 94,6 | 48,2 | 1,18 |
| 6,5 | 30,0 | 65,2 | 25,9 | 54,0 | 79,9 | 54,0 | 62,8 | 4,3 | 93,6 | 53,8 | 1,17 |
| 7,5 | 30,3 | 61,2 | 23,8 | 49,7 | 73,5 | 50,3 | 67,1 | 5,4 | 92,6 | 57,5 | 1,16 |
| 24 | 47,2 | 37,9 | 21,6 | 17,3 | 38,9 | 26,5 | 99,5 | 7,8 | 92,8 | 85,2 | 1,16 |
| Ü: Überstand, WF=Wiederfindung, Dowex: Dowex® WX8 | | | | | | | | | | | |

EP 1 392 621 B1

**Tab. 7: Entwicklung der Reinheiten von DHAEE und DPAEE im Überstand (Ethanol) während der Erwärmung auf unterschiedliche Temperaturen.**

| | 60°C | | 70°C | | 80°C | |
|---|---|---|---|---|---|---|
| t | DPAEE | DHAEE | DPAEE | DHAEE | DPAEE | DHAEE |
| [h] | [Area%] | [Area%] | [Area%] | [Area%] | [Area%] | [Area%] |
| 0,5 | 15,3 | 84,7 | 12,5 | 87,5 | 13,4 | 86,6 |
| 1 | 18,0 | 82,0 | 15,3 | 84,7 | 15,9 | 84,1 |
| 1,5 | 21,1 | 78,9 | 17,2 | 82,8 | 18,4 | 81,6 |
| 2 | 21,9 | 78,1 | 19,1 | 80,9 | 19,1 | 80,9 |
| 2,5 | 25,0 | 75,0 | 22,3 | 77,7 | 22,6 | 77,4 |
| Produktphase ACN | 5,0 | 95,0 | 4,6 | 95,4 | 4,9 | 95,3 |
| Ausgangsmaterial am Dowex 50 WX8 | 7,7 | 92,3 | 7,7 | 92,3 | 7,7 | 92,3 |

Dazu wird ein Pyrex-Röhrchen (Pyrex-Borosilicatgläser 16x100mm, Merck Eurolab, Darmstadt) jeweils mit 1 ml Ag-Dowex® 50 WX8 (200-400 mesh), der mit 1,21% DHAEE (g DHAEE/100 g H$^+$-Dowex)beladen wurde, befüllt und mit 2 ml Ethanol gemischt auf dem Vibromixer gemischt. Anschließend wird erwärmt auf 60, 70 und 80°C) und der Überstand gaschromatographisch analysiert. Die Reinheit des DHAEE am Dowex zu Beginn des Versuches betrug 92,3% (Dowex® 50 WX8, 200-400 mesh). Nach 2,5 h läßt man abkühlen, nimmt den Überstand ab und wäscht einmal mit 2 ml Ethanol. Anschließend wird der DHAEE mit 2 ml ACN für 3 h vom Dowex dekomplexiert.

Die gaschromatographische Analyse der ACN-Phase zeigt eine Anreicherung der DHAEE am mit Silber beladenen Kationentauscher auf ca. 95% in allen Versuchen.

Da die Ablösung des Produktes in vielen Fällen durch Acetonitril unerwünscht ist, wurden andere Verfahren erarbeitet, bei denen der Einsatz eines giftigen Lösungsmittel vermieden oder die Verwendung ganz ausgeschlossen werden kann. Im folgenden werden diese erläutert.

**Variante C Ablösen der Produkte vom Ag$^+$-Tauscher ohne ACN (Figur 1) (nicht erfindungsgemäß)**

In Falle der Komplexbindung mit PUFAs mit < 4 Doppelbindungen gelingt es schon durch Zugabe eines polaren Lösungsmittels (z.B. Ethanol bei Anlagerungen in unpolaren Lösungsmitteln wie n-Hexan) ohne oder mit gleichzeitiger Temperaturerhöhung (z.B. Wärmetauscher, Heizbad, Mikrowelle, Thermoblock) die PUFAs vom mit 100% Silber beladenen Kationentauscher abzulösen, ohne ACN zu verwenden.

In der Regel liegen die Temperaturen unmittelbar unter dem Siedepunkt des verwendeten Lösungsmittels. Sollte die PUFA zu stark komplexiert sein, kann ein Kationentauscher verwendet werden, der nicht zu 100% mit Silber beladen ist und demzufolge die PUFAs nicht so stark komplexieren kann. In vielen Fällen gelingt die Abtrennung der PUFAs schon durch die Verwendung eines der oben genannten Lösungsmitteln ohne Temperaturshift.

**Beispiel 9**

**Anlagerungsversuch eines Gemisches von DHAEE und DPAEE an 30% teilversilbertem Ag$^+$-Amberlyst® 15 (20-50 mesh)**

Dazu wird eine Mischung aus 0,63 g Docosahexaensäureethylester (DHAEE) und 0,17 g Docosapentaensäureethylester (ω-6 DPAEE) zu einer gerührten Lösung des zu 30% Silber-beladenen Tauschers (10 g H$^+$-Amberlyst®15) in 100 ml n-Hexan gegeben. Die Suspension wird bei 26 °C unter Schutzgas für 60 min bei 100 U/min geschüttelt.

Die Reinheit des Produktes kann ebenfalls so ermittelt werden (Tab. 8).

Die Reinheit des DHAEE wurde von 80% auf 90,5% gesteigert. Die Anlagerung ist bereits nach wenigen Minuten beendet. Die Beladung des Tauschers mit DHAEE beträgt 2,1% (g DHAEE/100 g H$^+$-Tauscher). Die theoretische Ausbeute aus der Tabelle beträgt an 90,5%-igem DHAEE liegt bei 32,5%

Ablösung des Produktes: nach Entfernen der Hexanphase mit dem nicht gebundenen Produkt wird der Tauscher mit 100 ml Ethanol versetzt, 30 min bei Raumtemperatur gerührt und die Ethanolphase abgenommen. Aus dieser Phase lassen sich bereits 176,3 mg DHAEE (Reinheit 87,6% Area) isolieren. Anschließend wird der Tauscher mit weiteren 100 ml Ethanol bei 70°C für 1 h mit 300 U/min gerührt. Es können aus dieser Phase weitere 51,6 mg DHAEE (94,5% Area) isoliert werden. Eine Fraktionierung der Ethanolphasen ist aber nicht notwendig. Aus der abgetrennten Hexanphase konnten nach Entfernen des Hexans 379,9 mg DHAEE reisoliert werden. Die Wiederfindung des gebundenen DHAEE liegt über 96%. Die vereinigten Ethanolextrake führen zu 227,9 mg DHAEE in einer Reinheit von 89,2% (Area). Die Ausbeute an DHAEE beträgt 32%.

**Tab. 8: Ergebnisse der Anlagerung eines Gemisches von DHAEE und DPAEE an teilversilbertem Ag⁺-Amberlyst® 15 (20-50 mesh)**

| Zeit | Ü DPAEE | Ü DHAEE | Ü DPAEE | Ü DHAEE | Summe | WF in EtOH | DHAEE Amberlyst | DPAEE Amberlyst | DHAEE Amberlyst | Ausbeute. DHAEE | Reinigungsfaktor DHAEE |
|---|---|---|---|---|---|---|---|---|---|---|---|
| (min) | (Area-%) | (Area-%) | (mg) | (mg) | (mg) | (%) | (mg) | (mg) | (Area-%) | (%) | |
| 0 | 19,2 | 71,5 | 161,7 | 601,3 | 763,0 | 95,4 | 29,4 | 7,7 | 79,2 | 4,7 | |
| 10 | 22,3 | 65,8 | 141,4 | 416,9 | 558,1 | 69,8 | 213,8 | 28,1 | 88,4 | 33,9 | 1,11 |
| 30 | 22,9 | 66,0 | 151,1 | 435,5 | 586,7 | 73,3 | 195,2 | 18,3 | 91,5 | 30,9 | 1,14 |
| 60 | 22,8 | 65,7 | 147,9 | 425,6 | 573,5 | 71,7 | 205,1 | 21,5 | 90,5 | 32,5 | 1,13 |
| Ü: Überstand; WF: Wiederfindung,;Amberlyst: Amberlyst® 15 | | | | | | | | | | | |

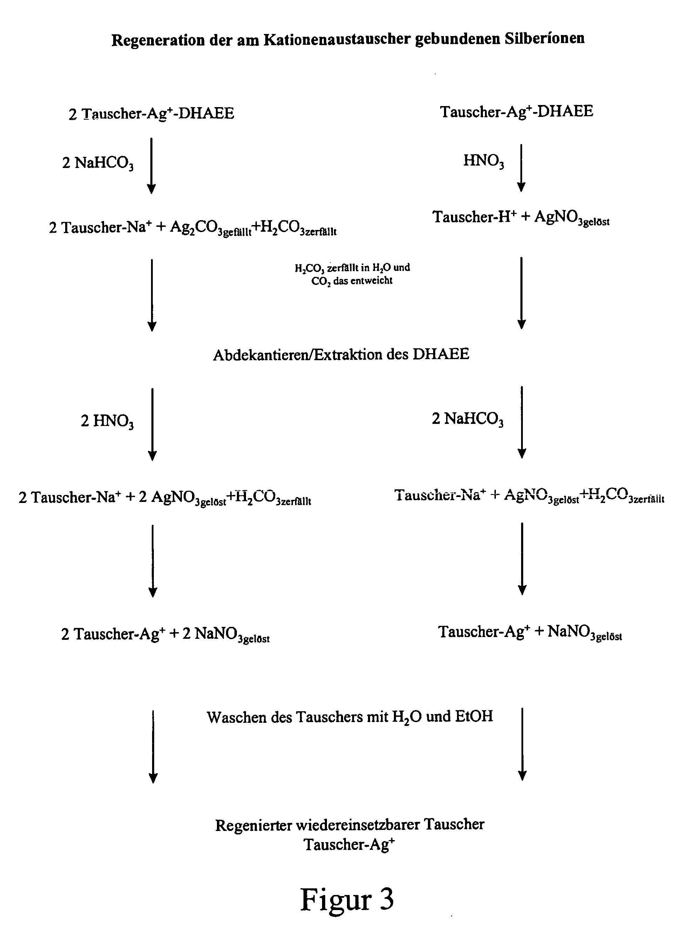

**Variante B Ablösen des Silbers vom Kationentauscher mit gleichzeitiger Freisetzung des Fettsäureethylesters (Figuren 1 und 3) (erfindungsgemäß)**

**Beispiel 10**

**Regenerative Ablösung des Fettsäureethylesters**

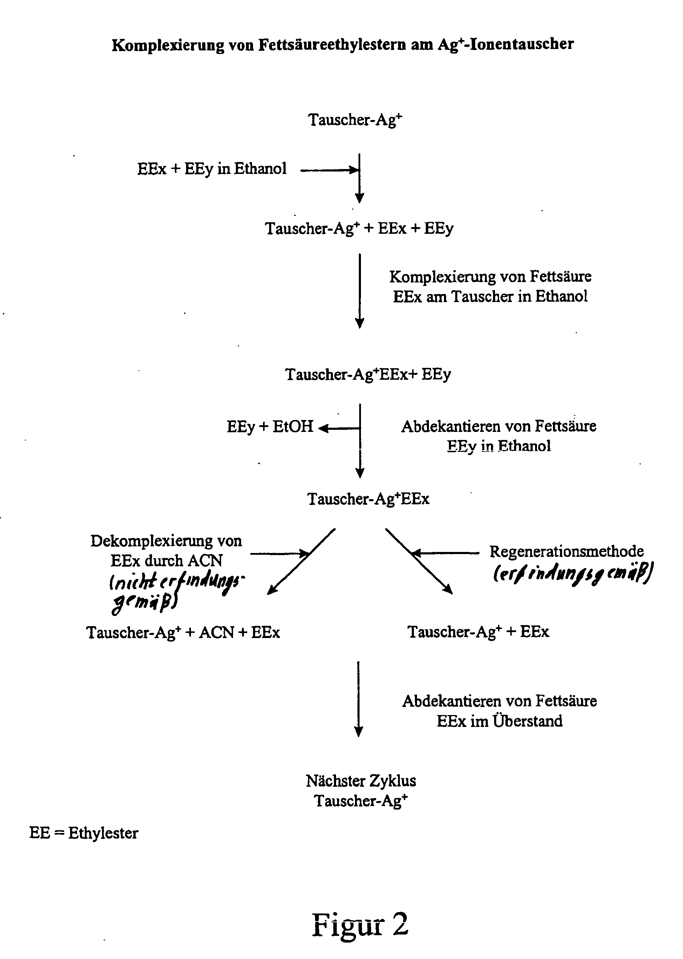

Um die Verwendung von Acetonitril bei der Ablösung des DHAEE vom Ag$^+$-Dowex® zu vermeiden, können die Prozesse wie in Figur 2 beschrieben verwendet werden.

Nach Abtrennen des ethanolischen Überstandes aus der Reaktion wird der mit Silber beladenen Kationentauscher mit einem Überschuß 0,4 M Natriumnitrat-Lösung (NaNO$_3$) silberfrei gewaschen. Dies kann im Rundkolben (Batch) oder in einer Glassäule erfolgen (kontinuierlich). Gleichzeitig löst sich der DHAEE vom Kationentauscher und kann als ölige Phase aus der wäßrigen Phase abdekantiert werden. Dabei ist es nicht erforderlich, das gesamte Silber loszulösen, um den DHAEE abzulösen. In der Praxis stellt sich die vollständige Ablösung jedoch als einfachste Methode dar.

Nach Abtrennen des Produktes wird das Silber mit einer Karbonat-Lösung ausgefällt (z.B. Na$_2$CO$_3$, K$_2$CO$_3$ oder NaHCO$_3$). Das gebildete Silberkarbonat (z.B. Ag$_2$CO$_3$) fällt als gelber Niederschlag aus und kann in einfachster Weise vom Überstand abfiltriert werden. Anschließend wird das Silberkarbonat mit stöchiometrischen Mengen Salpetersäure (HNO$_3$) in Lösung gebracht. Diese Lösung kann zur Wiederanlagerung des Silbers direkt verwendet werden.

Die Beladung des Tauschers mit Silber entspricht der Ausgangsbeladung. Die Selektivität des Tauschers bleibt voll erhalten.

Auf diesem Wege kann die Ablösung und Isolierung des DHAEE ohne Lösungsmittel erfolgen, was besonders für Anwendungen in Lebensmitteln von enormen Vorteil ist.

**Patentansprüche**

1. Verfahren zur Abtrennung einer oder mehrerer am stärksten ungesättigten Verbindung(en) aus einer flüssigen Mischung, umfassend zusätzlich eine oder mehrere weniger stark ungesättigte organische Verbindung(en) und/ oder eine oder mehrere gesättigte organische Verbindung(en), **dadurch gekennzeichnet, dass** man

   (i) einen sauren Kationenaustauscher mit Silberionen belädt,
   (ii) den beladenen Kationenaustauscher mit der flüssigen Mischung mischt,
   (iii) die Mischung im Batch-Verfahren bei einer Temperatur rührt, die unterhalb des Siedepunktes des verwendeten Lösungsmittels liegt,
   (iv) den Überstand abtrennt, und
   (v) die am stärksten ungesättigte(n) Verbindung(en) vom Kationenaustauscher ablöst,

   wobei

   ➢ die flüssige Mischung Roh-Estergemische, prozessierte Öle, Estergemische, Fettsäuregemische und/oder Derivate von mehrfach ungesättigten Fettsäuren umfasst,
   ➢ man die am stärksten ungesättigte (n) Verbindung (en) vom Kationenaustauscher ablöst, indem durch Zugabe von Natriumnitrat das Silber von dem Ionentauscher abgelöst wird oder indem man das Silber durch Zugabe einer Säure oder Base ablöst.

2. Verfahren nach Anspruch 1, **dadurch gekennzeichnet, dass** die am stärksten ungesättigte Verbindung eine mehrfach ungesättigte Fettsäure mit mindestens zwei Doppelbindungen ist.

3. Verfahren nach einem der vorstehenden Ansprüche, **dadurch gekennzeichnet, dass** die flüssige Mischung ein Lösungsmittel umfasst.

4. Verfahren nach Anspruch 3, **dadurch gekennzeichnet, dass** das Lösungsmittel ausgewählt ist aus der Gruppe der Ether, Ester, Ketone, Alkane, Alkohole und Nitrile.

5. Verfahren nach Anspruch 3, **dadurch gekennzeichnet, dass** das Lösungsmittel ausgewählt ist aus der Gruppe umfassend n-Hexan, n-Heptan, n-Pentan, Ethanol, Methanol und Acetonitril.

6. Verfahren nach einem der vorstehenden Ansprüche, **dadurch gekennzeichnet, dass** die flüssige Mischung eine gesättigte Fettsäure und/oder eine ungesättigte Fettsäure mit n Doppelbindungen und/oder eine Fettsäure mit n+1 oder mehr Doppelbindungen umfasst, wobei die Fettsäure mit n+1 oder mehr Doppelbindungen von der gesättigten bzw. der ungesättigten Fettsäure mit n Doppelbindungen getrennt wird, wobei n für eine ganze Zahl zwischen 1 und 10 steht.

**7.** Verfahren nach einem der vorstehenden Ansprüche, **dadurch gekennzeichnet, dass** die flüssige Mischung Fischöle, Pflanzenöle und/oder mikrobielle Öle umfasst.

**8.** Verfahren nach einem der Ansprüche 2 bis 7, **dadurch gekennzeichnet, dass** die mehrfach ungesättigte Fettsäure ausgewählt ist aus der Gruppe umfassend Hexadecadiensäuren, Hexadecatriensäuren, Hexadecatetraensäuren, Linolsäure, y-Linolensäure, a-Linoiensäure, Stearidonsäure, Arachidonsäure, Eicosatriensäuren, Eicosatetraensäuren, Eicosapentaensäuren, Docosapentaensäuren, Docosahexaensäure, Tetracosadiensäuren, Octacosaoctaensäuren.

**9.** Verfahren nach einem der vorstehenden Ansprüche, **dadurch gekennzeichnet, dass** der saure Kationenaustauscher ausgewählt ist aus der Gruppe umfassend Dowex® 50 WX8, Dowex® 50 WX4, Dowex® 50 WX2, Dowex® MWC1, Dowex® MSC1, Dowex® Monosphere C-350, Dowex® CCR-2, Dowex® DR 2030, Amberlite® CG50, Amberlite® IR-120, Amberlyst® 15, Bio-Rex® 70 Resin, Macherey & Nagel PS-DVB®.

**10.** Verfahren nach einem der vorstehenden Ansprüche, **dadurch gekennzeichnet, dass** man den sauren Kationentauscher durch Behandeln mit Silbernitrat belädt.

**11.** Verfahren nach einem der vorstehenden Ansprüche, **dadurch gekennzeichnet, dass** man die Mischung aus beladenem Kationenaustauscher mit der flüssigen Mischung bei erhöhter Temperatur rührt

**12.** Verfahren nach Anspruch 11, **dadurch gekennzeichnet, dass** die Temperaturerhöhung durch Einwirken einer externen Wärmequelle, von Mikrowellen, Ultraschall oder elektromagnetischer Strahlung bewirkt wird.

**13.** Verfahren nach einem der Ansprüche 1 bis 12, **dadurch gekennzeichnet, dass** die flüssige Mischung mindestens eine mehrfach ungesättigte Fettsäure mit mindestens 4 Doppelbindungen umfasst und man den sauren Kationenaustauscher vollständig mit Silberionen belädt und die am stärksten ungesättigte (n) Verbindung (en) durch Ablösen der Silberionen von dem Kationenaustauscher ablöst.

**14.** Verfahren nach Anspruch 13, **dadurch gekennzeichnet, dass** die Ablösung der Silberionen von dem Kationenaustauscher durch Zugabe von Natriumnitrat erfolgt.

**Claims**

**1.** A method for the separation of one or more of the most highly unsaturated compound(s) from a liquid mixture, comprising additionally one or more less highly unsaturated organic compound(s) and/or one or more saturated organic compound(s), **characterized in that**

(i) an acidic cation exchanger is loaded with silver ions,
(ii) the loaded cation exchanger is mixed with the liquid mixture,
(iii) the mixture is stirred in a batch method at a temperature which is lower than the boiling point of the solvent used,
(iv) the supernatant is separated, and
(v) the most highly unsaturated compound(s) is (are) removed from the cation exchanger,

in which

➢ the liquid mixture comprises raw ester mixtures, processed oils, ester mixtures, fatty acid mixtures and/or derivatives of polyunsaturated fatty acids,
➢ the most highly unsaturated compound(s) is (are) removed from the cation exchanger by removing the silver from the ion exchanger by addition of sodium nitrate or by removing the silver by addition of an acid or base.

**2.** The method according to claim 1, **characterized in that** the most highly unsaturated compound is a polyunsaturated fatty acid having at least two double bonds.

**3.** The method according to any one of the preceding claims, **characterized in that** the liquid mixture comprises a solvent.

**4.** The method according to claim 3, **characterized in that** the solvent is selected from the group of ethers, esters, ketones, alkanes, alcohols and nitriles.

**5.** The method according to claim 3, **characterized in that** the solvent is selected from the group comprising n-hexane, n-heptane, n-pentane, ethanol, methanol and acetonitrile.

**6.** The method according to any one of the preceding claims, **characterized in that** the liquid mixture comprises a saturated fatty acid and/or an unsaturated fatty acid having n double bonds and/or a fatty acid having n+1 or more double bonds, wherein the fatty acid having n+1 or more double bonds is separated from the saturated or unsaturated fatty acid having n double bonds, wherein n represents an integer between 1 and 10.

**7.** The method according to any one of the preceding claims, **characterized in that** the liquid mixture comprises fish oils, vegetable oils and/or microbial oils.

**8.** The method according to any one of claims 2 to 7, **characterized in that** the polyunsaturated fatty acid is selected from the group comprising hexadecadiene acids, hexadecatriene acids, hexadecatetraene acids, linoleic acid, γ-linolenic acid, α-linolenic acid, stearidonic acid, arachidonic acid, eicosatrienoic acids, eicosatetraenoic acids, eicosapentaenoic acids, docosapentaenoic acids, docosahexaenoic acids, tetracosadienoic acids, octacosaoctaenoic acids.

**9.** The method according to any one of the preceding claims, **characterized in that** the acidic cation exchanger is selected from the group comprising Dowex® 50 WX8, Dowex® 50 WX4, Dowex® 50 WX2, Dowex® MWC1, Dowex® MSC1, Dowex® Monosphere C-350, Dowex® CCR-2, Dowex® DR 2030, Amberlite® CG50, Amberlite® IR-120, Amberlyst® 15, Bio-Rex® 70 Resin, Macherey & Nagel PS-DVB®.

**10.** The method according to any one of the preceding claims, **characterized in that** the acidic cation exchanger is loaded by treating it with silver nitrate.

**11.** The method according to any one of the preceding claims, **characterized in that** the mixture of loaded cation exchanger with the liquid mixture is stirred at an increased temperature.

**12.** The method according to claim 11, **characterized in that** the temperature increase is effected by action of an external heat source, of microwaves, ultrasound or electromagnetic radiation.

**13.** The method according to any one of claims 1 to 12, **characterized in that** the liquid mixture comprises at least one polyunsaturated fatty acid having at least 4 double bonds and the acidic cation exchanger is completely loaded with silver ions and the most highly unsaturated compound(s) is (are) removed by removing the silver ions from the cation exchanger.

**14.** The method according to claim 13, **characterized in that** the removal of the silver ions from the cation exchanger is effected by addition of sodium nitrate.

**Revendications**

**1.** Procédé de séparation d'un ou plusieurs composé(s) le(s) plus fortement non saturés à partir d'un mélange liquide, comprenant en plus, un ou plusieurs composé(s) organique(s) moins fortement non saturé(s) et/ou un ou plusieurs composé(s) organiques(s) saturé (s), **caractérisé en ce que** l'on

(i) charge un échangeur de cations acide d'ions argent,
(ii) mélange l'échangeur de cations chargé avec le mélange liquide,
(iii) agite le mélange dans procédé batch à une température qui se trouve en dessous du point d'ébullition du solvant utilisé,

(iv) sépare le surnageant, et

(v) on enlève le(s) composé(s) le(s) plus fortement non saturé(s) de l'échangeur de cations.

sachant que

→ le mélange fluide comprend des mélanges d'ester brut, des huiles traitées, des mélanges d'ester, des mélanges d'acides gras et/ou des dérivés d'acides gras non saturés de façon multiple,

→ on enlève le(s) composé(s) plus fortement non saturé(s) de l'échangeur de cations, en enlevant l'argent de l'échangeur d'ions par ajout de nitrate de sodium ou en enlevant l'argent par ajout d'un acide ou d'une base.

2. Procédé selon la revendication 1, **caractérisé en ce que** le composé le plus fortement non saturé est un acide gras non saturé de façon multiple avec au moins deux double liaisons.

3. Procédé selon l'une des revendications précédente **caractérisé en ce que** le mélange liquide comprend un solvant.

4. Procédé selon la revendication 3 **caractérisé en ce que** le solvant est sélectionné dans le groupe des éthers, des esters, des cétones, des alcanes, des alcools et des nitriles.

5. Procédé selon la revendication 3, **caractérisé en ce que** le solvant est sélectionné dans le groupe comprenant hexane-n, heptane-n, pentane-n, éthanol, méthanol et acétonitrile.

6. Procédé selon l'une des revendications précédentes **caractérisé en ce que** le mélange liquide comprend un acide gras saturé et/ou un acide gras non saturé avec doubles liaisons n et/ou un acide gras avec des liaisons doubles n+1 ou plus, sachant que l'acide gras avec des liaisons doubles n+1 ou plus est séparé de l'acide gras saturé ou non saturé avec des liaisons doubles n, sachant que n est un nombre entier situé entre 1 et 10.

7. Procédé selon l'une des revendications précédentes, **caractérisé en ce que** le mélange liquide comprend des huiles de poisson, des huiles végétales et/ou des huiles microbiennes.

8. Procédé selon l'une des revendications de 2 à 7, **caractérisé en ce que** l'acide gras non saturé de façon multiple est sélectionné dans le groupe comprenant des acides hexadécanoïques, acides hexadecatriénoïques, acides hexadécatétraénoïques, de l'acide linoléique, acide linolénique-y, acide linolénique-a, acide stéaridonique, acide arachidonique, des acides eicosatriénoïques, acides eicosatétraénoïques, acides eicosapentaénoïques, acides docosapentaénoïques, d'acide docosahexaénoïque, des acides tétracosanoïques, acides octacosaoctaénoïques.

9. Procédé selon l'une des revendications précédentes, **caractérisé en ce que** l'échangeur de cations acide est sélectionné dans le groupe comprenant Dowex® 50 WX8, Dowex® 50 WX4. Dowex® 50 WX2, Dowex® MWC1, Dowex® MSC1, Dowex® Monosphere C-350, Dowex® CCR-2, Dowex® DR2030, Amberlite® CG50, Amberlite® IR-120, Amberlyst® 15, Bio-Rex® 70 Resin, Macherey & Nagel PS-DVB®.

10. Procédé selon l'une des revendications précédentes **caractérisé en ce que** l'on charge l'échangeur de cations acide par traitement avec nitrate d'argent.

11. Procédé selon l'une des revendications précédentes **caractérisé en ce que** l'on agite le mélange provenant de l'échangeur de cations chargé avec le mélange liquide à température élevée.

12. Procédé selon revendication 11 **caractérisé en ce que** l'augmentation de température est provoquée par une source de chaleur externe, par des micro-ondes, des ultrasons ou un rayonnement électromagnétique.

13. Procédé selon l'une des revendications 1 à 12, **caractérisé en ce que** le mélange liquide comprend au moins un acide gras non saturé de façon multiple avec au moins 4 liaisons doubles et que l'on charge l'échangeur de cations acide entièrement avec des ions argent et on enlève le(s) composé (s) les plus fortement non saturé (s) en retirant les ions argent de l'échangeur de cations.

14. Procédé selon revendication 13 **caractérisé en ce que** le décollement des ions argent de l'échangeur de cations s'effectue par ajout de nitrate de sodium.

**Prinzip der Aufreinigung von PUFAs durch selektive Extraktion
vom Ag⁺-Ionentauscher**

# Figur 1

EP 1 392 621 B1

**Komplexierung von Fettsäureethylestern am Ag⁺-Ionentauscher**

Tauscher-Ag⁺

EEx + EEy in Ethanol $\longrightarrow$

Tauscher-Ag⁺ + EEx + EEy

Komplexierung von Fettsäure
EEx am Tauscher in Ethanol

Tauscher-Ag⁺EEx+ EEy

EEy + EtOH $\longleftarrow$  Abdekantieren von Fettsäure
EEy in Ethanol

Tauscher-Ag⁺EEx

Dekomplexierung von
EEx durch ACN $\longrightarrow$
*(nicht erfindungsgemäß)*

Regenerationsmethode
*(erfindungsgemäß)*

Tauscher-Ag⁺ + ACN + EEx

Tauscher-Ag⁺ + EEx

Abdekantieren von Fettsäure
EEx im Überstand

Nächster Zyklus
Tauscher-Ag⁺

EE = Ethylester

# Figur 2

## Regeneration der am Kationenaustauscher gebundenen Silberionen

2 Tauscher-Ag$^+$-DHAEE                    Tauscher-Ag$^+$-DHAEE

2 NaHCO$_3$ $\downarrow$                    HNO$_3$ $\downarrow$

2 Tauscher-Na$^+$ + Ag$_2$CO$_{3\text{gefällt}}$+H$_2$CO$_{3\text{zerfällt}}$          Tauscher-H$^+$ + AgNO$_{3\text{gelöst}}$

$\downarrow$          H$_2$CO$_3$ zerfällt in H$_2$O und CO$_2$ das entweicht          $\downarrow$

Abdekantieren/Extraktion des DHAEE

2 HNO$_3$ $\downarrow$                    2 NaHCO$_3$ $\downarrow$

2 Tauscher-Na$^+$ + 2 AgNO$_{3\text{gelöst}}$+H$_2$CO$_{3\text{zerfällt}}$          Tauscher-Na$^+$ + AgNO$_{3\text{gelöst}}$+H$_2$CO$_{3\text{zerfällt}}$

$\downarrow$                    $\downarrow$

2 Tauscher-Ag$^+$ + 2 NaNO$_{3\text{gelöst}}$          Tauscher-Ag$^+$ + NaNO$_{3\text{gelöst}}$

$\downarrow$ Waschen des Tauschers mit H$_2$O und EtOH $\downarrow$

Regenierter wiedereinsetzbarer Tauscher
Tauscher-Ag$^+$

# Figur 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- JP 45021376 B
- JP 06279781 B

- US 4721584 A


**In der Beschreibung aufgeführte Nicht-Patentliteratur**

- **Adlof, R. O. ; E.A. Emken.** The isolation of omega-3 polyunsaturated fatty acids and methyl esters of fish oil by silver resin chromatography. *JAOCS,* 1985, vol. 62 (11), 1592-1595
- **Christie, W.W.** A stable silver loaded column for the separation of lipids by high performance liquid chromatography. *J. of High resolution Chromatography & Chromatography Communications,* 1987, vol. 10, 148-150
- **Kadota, Yasuhiko ; Tanaka, Isao ; Ohtsu, Yutaka ; Yamaguchi, Michihiro.** Separation of polyunsaturated fatty acids by chromatography using a silver-loaded spherical clay. II Industrial-scale preparation of high purity DHA. *Nihon Yyukagakkaaishi,* 1998, vol. 47 (4), 351-357
- **Nieto, S. ; Ana M Cordoba ; J. Sanhueza ; A. Velenzueela.** Obtention of highly purified fractions of eicosapentaenoic acid and docosahexaenoic acid from sardine oil by silver-resin chromatography: A semi-preparative procedure. *Grasas y Aceites,* 1997, vol. 48 (4), 197-199
- **Belarbi, El Hassan ; Emilio Molina ; Yusuf Chisti.** A process for high yield and scalable recovery of high purity eicosapentaenoic acid esters from microalgae. *Enzyme and Microbial Technology,* 2000, vol. 26, 516-529
- **Pyell, U. ; S. Schober ; G. Stork.** Ligand-exchange chromatographic separation of polycyclic aromatic hydrocarbons and polycyclic aromatic sulfur heterocycles on a chelating silic gel loaded with palladium (II) or silver (I) cations. *Fresenius J Anal Chem.,* 1997, vol. 359, 538-541
- **Janak, K. ; M. Demirbueker ; I. Haegglund ; L.G. Blomberg.** Modifications of poly(methyl-3-propylthiol)siloxane to give stationary phases for open tubular supercritical fluid chromatography. *Chromatographia,* 1992, vol. 34 (5-8), 335-341
- **Adlof, R.O. ; H. Rakoff ; E. A. Emken.** Partial Argentation Resin Chromatography (PARC): I. Effect of Percent Silver on Elution and Separation of Methyl Octadecadienoate Isomers. *JAOCS,* 1980, vol. 9, 273-275

- **DeJarlais, W. J. ; R. O. Adlof ; E. A. Emken.** Acetonitrile as Eluent in Silver Resin Column Chromatography. *JAOCS,* 1983, vol. 60 (5), 975-978
- **Alkio, M. ; C. Gonzalez ; M. Jäntti ; O. Altonen.** Purification of polyunsaturated fatty acid esters from Tuna oil with supercritical fluid chromatography. *JAOCS,* 2000, vol. 77 (3), 315-321
- **Tanaka ; Isao.** Supercritical chromatography facilitates fatty acid production. *Chem. Eng.,* 19. April 1996
- **Yamamura, R. ; Y. Shimomura.** Industrial highperformance liquid chromatography purification of docosahexaenoic acid ethyl ester and docosapentaenoic acid ethyl ester from single cell oil. *JAOCS,* 1997, vol. 74 (11), 1435-1440
- **Zhou, Dequan ; Xuebing Xu.** Enzymatic enrichment of Long-chain Polyunsaturated Fatty Acids from Fish Oils. *Shipin Kexue,* 2000, vol. 21 (12), 188-194
- **Teramoto, M. ; H. Matsuyama ; N. Ohnishi ; S. Uwagawa ; K. Nakai.** Extraction of ethyl and methyl esters of polyunsaturated fatty acids with aqueous silver nitrate solutions. *Ind. Eng. Chem. Res.,* 1994, vol. 33, 341-345
- **Suzuki, T. ; S. Kikuchi ; K. Nakano ; S. Kato ; K. Nagahama.** Supercritical fluid extraction of polyunsaturated fatty acid ethyl esters from aqueous silver nitrate solution. *Bioseparation,* 1993, vol. 3, 197-204
- **Wilson, R. ; J. R. Henderson ; I. Burkow ; J. R. Sargent.** The enrichment of n-3 polyunsaturated fatty acids, using aminopropyl solid phase extraction columns. *Lipids,* 1993, vol. 28 (1), 51-54
- **Shibaki, A. ; Y. Irimoto ; K. Saito ; K. Sugita ; T. Baba ; I. Honjyo ; S. Moriyama ; T. Sugo.** Selective Binding of Docosahexaenoic acid ethyl ester to a silver ion-loaded porous hollow-fiber membrane. *JAOCS,* 1999, vol. 76 (7), 771-775
- **Yokochi et al.** Optimization of docosahexaenoic acid production by Schizochytrium limacinum SR21. *Appl. Microbiol. Biotechnol.,* 1998, vol. 49, 72-76

- **Nieto, S. ; A. M. Cordoba ; J. Sanhuenzy ; A. Valenzuela.** Obtention of highly purified fractions of eicosapentaenoic acid and docosahexaenoic acid from sardine oil by silver-resin chromatography: A semi-preparative procedure. *Grasas y Aceites,* 1997, vol. 48 (4), 197-199

- **Nieto, S. ; A. M. Cordoba ; J. Sanhuenzy ; A. Valenzuela.** Obtention of highly purified fractions of eicosapentaenoic acid and docosahexaenoic acid from sardine oil by silver-resin chromatography: A semi-preparative procedure. *Grasas y Aceites,* 1997, vol. 48 (4), 197-199